(19) Europäisches Patentamt
European Patent Office
Office européen des brevets

(11) EP 1 973 569 B1

(12) EUROPEAN PATENT SPECIFICATION

(45) Date of publication and mention
of the grant of the patent:
**22.05.2013 Bulletin 2013/21**

(51) Int Cl.:
*A61K 45/06* (2006.01)   *A61K 38/08* (2006.01)
*A61K 31/4188* (2006.01)   *A61K 41/00* (2006.01)
*A61P 35/00* (2006.01)

(21) Application number: **07718269.9**

(22) Date of filing: **18.01.2007**

(86) International application number:
**PCT/US2007/001446**

(87) International publication number:
**WO 2007/084670 (26.07.2007 Gazette 2007/30)**

(54) **SPECIFIC THERAPY USING INTEGRIN LIGANDS FOR TREATING CANCER**

SPEZIFISCHE THERAPIE MIT INTEGRIN-LIGANDEN ZUR KREBSBEHANDLUNG

TRAITEMENT SPECIFIQUE UTILISANT DES LIGANDS DE INTEGRINE DESTINE A TRAITER UN CANCER

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.01.2006 EP 06000988**
**18.01.2006 EP 06001044**
**20.01.2006 EP 06006003**
**31.07.2006 EP 06015883**

(43) Date of publication of application:
**01.10.2008 Bulletin 2008/40**

(60) Divisional application:
**10015608.2 / 2 335 733**
**10015609.0 / 2 338 518**

(73) Proprietor: **Merck Patent GmbH**
**64293 Darmstadt (DE)**

(72) Inventors:
• **GOODMAN, Simon**
**64347 Griesheim (DE)**
• **PICARD, Martin, Andreas**
**64291 Darmstadt (DE)**
• **MIKKELSEN, Tom**
**West Bloomfield, MI 48323 (US)**
• **NIPPGEN, Johannes**
**D-64297 Darmstadt (DE)**
• **GRIMM, Ulrike**
**83607 Holzkirchen (DE)**
• **STUPP, Roger**
**CH-1012 Lausanne (CH)**
• **WELLER, Michael**
**D-72070 Tuebingen (DE)**
• **HARSTRICK, Andreas**
**64397 Modautal (DE)**
• **GRELL, Matthias**
**64291 Darmstadt (DE)**

(56) References cited:
**WO-A-02/055106**

• **BURKE P A ET AL: "Cilengitide Targeting of alphavbeta3 Integrin Receptor Synergizes with Radioimmunotherapy to Increase Efficacy and Apoptosis in Brest Cancer Xenografts1" CANCER RESEARCH, AMERICAN ASSOCIATION FOR CANCER RESEARCH, BALTIMORE, MD, US, vol. 62, no. 15, 1 August 2002 (2002-08-01), pages 4263-4272, XP002903574 ISSN: 0008-5472**
• **LODE H N ET AL: "Synergy between an antiangiogenic integrin alphav antagonist and an antibody-cytokine fusion protein eradicates spontaneous tumor metastases" PROCEEDINGS OF THE NATIONAL ACADEMY OF SCIENCES OF USA, NATIONAL ACADEMY OF SCIENCE, WASHINGTON, DC, US, vol. 96, no. 4, 16 February 1999 (1999-02-16), pages 1591-1596, XP002134006 ISSN: 0027-8424**

- STOELTZING OLIVER ET AL: "Inhibition of integrin alpha5beta1 function with a small peptide (ATN-161) plus continuous 5-FU infusion reduces colorectal liver metastases and improves survival in mice." INTERNATIONAL JOURNAL OF CANCER. JOURNAL INTERNATIONAL DU CANCER 20 APR 2003, vol. 104, no. 4, 20 April 2003 (2003-04-20), pages 496-503, XP002439488 ISSN: 0020-7136
- ABDOLLAHI AMIR ET AL: "Inhibition of alpha(v) beta3 integrin survival signaling enhances antiangiogenic and antitumor effects of radiotherapy." CLINICAL CANCER RESEARCH : AN OFFICIAL JOURNAL OF THE AMERICAN ASSOCIATION FOR CANCER RESEARCH 1 SEP 2005, vol. 11, no. 17, 1 September 2005 (2005-09-01), pages 6270-6279, XP002439489 ISSN: 1078-0432
- STUPP ROGER ET AL: "Radiotherapy plus concomitant and adjuvant temozolomide for glioblastoma." THE NEW ENGLAND JOURNAL OF MEDICINE 10 MAR 2005, vol. 352, no. 10, 10 March 2005 (2005-03-10), pages 987-996, XP002439490 ISSN: 1533-4406 cited in the application

## Description

### Technical Field of the Invention:

The invention relates to a specific therapy form for the treatment of astrocytoma comprising at least one integrin ligand, selected from cyclo-(ARG-GLY-ASP-DPHE-NMEVAL) and the pharmaceutically acceptable solvates and salts thereof, together with the cancer cotherapeutic agent tepiozolomide and radiotherapy that have synergistic efficacy when administered together with said integrin ligand, preferably in a timely controlled manner. The therapy will result in a synergistic potential increase of the inhibition effect of each individual therapeutic on tumor cell and tumor endothelial cell proliferation, yielding more effective treatment than found by administering an individual component alone, together or in another therapy regime but the regime of the present invention.

### Background of the Invention:

Vascular endothelial cells are known to contain at least three RGD-dependent integrins, including the vitronectin receptors $\alpha_v\beta_3$ or $\alpha_v\beta_5$ as well as the collagen types I and IV receptors $\alpha_v\beta_1$ and $\alpha_2\beta_1$, the laminin receptors $\alpha_6\beta_1$ and $\alpha_3\beta_1$, and the fibronectin receptor $\alpha_5\beta_1$ (Davis et al., 1993, J. Cell. Biochem. 51, 206). The smooth muscle cell is known to contain at least six RGD-dependent integrins, including $\alpha_v\beta_3$ and $\alpha_v\beta_5$.
Inhibition of cell adhesion in vitro using monoclonal antibodies immunospecific for various integrin $\alpha$ or $\beta$ subunits have implicated the vitronectin receptor $\alpha_v\beta_3$ in cell adhesion processes of a variety of cell types including microvascular endothelial cells (Davis et al., 1993, J. Cell. Biol. 51, 206).
Integrins are a class of cellular receptors known to bind extracellular matrix proteins, and mediate cell-extracellular matrix and cell-cell interactions, referred generally to as cell adhesion events. The integrin receptors constitute a family of proteins with shared structural characteristics of non-covalenty associated heterodimeric glycoprotein complexes formed of $\alpha$ and $\beta$ subunits. The vitronectin receptor, named for its original characteristic of preferential binding to vitronectin, is now known to refer to four different integrins, designated $\alpha_v\beta_1$, $\alpha_v\beta_3$, $\alpha_v\beta_5$ and $\alpha_v\beta_8$. $\alpha_v\beta_1$ binds fibronectin and vitronectin. $\alpha_v\beta_3$ binds a large variety of ligands, including fibrin, fibrinogen, laminin, thrombospondin, vitronectin and von Willebrand's factor. $\alpha_v\beta_5$ binds vitronectin. It is clear that there are different integrins with different biological functions as well as different integrins and subunits having shared biological specificity and function. One important recognition site in a ligand for many integrins is the Arg-Gly-Asp (RGD) tripeptide sequence. RGD is found in all of the ligands identified above for the vitronectin receptor integrins. The molecular basis of RGD recognition by $\alpha_v\beta_3$ has been identified (Xiong *et al.*, 2001) This RGD recognition site can be mimicked by linear and cyclic (poly)peptides that contain the RGD sequence. Such RGD peptides are known to be inhibitors or antagonists, respectively, of integrin function. It is important to note, however, that depending upon the sequence and structure of the RGD peptide, the specificity of the inhibition can be altered to target specific integrins. Various RGD polypeptides of varying integrin specificity have been described, for example, by Cheresh, et al., 1989, Cell 58, 945, Aumailley et al., 1991, FEBS Letts. 291, 50, and in numerous patent applications and patents (e.g. US patents 4,517,686, 4,578,079, 4,589,881, 4,614,517, 4,661,111, 4,792,525; EP 0770 622).
The generation of new blood vessels, or angiogenesis, plays a key role in the growth of malignant disease and this has generated much interest in developing agents that inhibit angiogenesis.
Nevertheless, although various combination therapies utilizing potential angiogenesis inhibitors are under investigation, in clinical trials and on the market, the outcome of these therapies are not fruitful enough. Therefore, there still exists a need in the art to develop further combinations which can show increased efficacy and reduced side-effects.
It is known today that tumor vasculature is different from vasculature of healthy tissue. The vasculature is characteristic for the tumor and distinct from the stable, dormant vasculature of healthy tissue. It is often characterized by an increased expression and priming of specific cell adhesion molecules of the alpha-v-integrin series, especially $a_v\beta_3$ and $\alpha_v\beta_5$. When activated these integrins enhance the cellular response to growth factors that drive angiogenesis, for example VEGFA and FGF2: VEGFA was originally termed vascular permeability factor, and it acts via the SRC kinase pathway to increase local vascular permeability. VEGRF2, when activated, increases the activity of $\alpha_v\beta_3$ integrin.
Further, solid tumors depend on an induced and cooped vasculature from the host to develop. This vasculature has unusual molecular properties that distinguish it from the normal host vasculature: it tends to be activated, i.e. progressing through cell cycle under the influence of tumor-derived factors like VEGFs, FGFs and others, and expresses endothelial activation markers like ICAM, VCAM and alpha-v-series Integrins, e.g. $\alpha_v\beta_3$ and $\alpha_v\beta_5$, in a ligand competent state. It has a defective extracellular matrix, and is classically described as leaky. It is notable that tumors often resist therapies systemically applied via the blood stream, due to abnormal nature of tumor vasculature.
The metastatic process is a multistep event and represents the most dreadful aspect of cancer. At the moment of diagnosis, cancers are frequently far advanced in their natural history, and the presence of metastases is a common event. In fact, approximately 30% of patients have detectable metastases at the moment of clinical diagnosis and a further 30% of patients have occult metastases. Metastases can be disseminated and they can infest different organs at the same time, or localize to a specific organ. In the case of localized disease, surgery is the treatment of choice; however recurrence and prognosis depend on many criteria such as: resectability, patient's clinical situation, and number of metastases.

After resection, recurrence is common, suggesting that micrometastatic foci are present at the moment of diagnosis. Systemic chemotherapy is an ideal setting but only few patients are cured by it, and in the majority systemic chemotherapy fails. Many physiological barriers and pharmacokinetic parameters contribute to decrease its efficacy.

Liver, lungs and lymph nodes are filtration organs and therefore inclined to metastasization. The poor chemosensitivity of metastases, peculiarly those of colorectal origin has forced many researchers to use methods for increasing the time and the concentration of drugs. The need for decreasing or limiting the side effects for this important and delicate organ led to the development of the technique of liver isolation for perfusion of antineoplastic agents. (K. R. Aigner, Isolated liver perfusion. In: Morris DL, McArdle CS, Onik GM, eds. Hepatic Metastases. Oxford: Butterworth Heinemann, 1996. 101-107). Since 1981, modifications and technical improvements have been continuously introduced. Liver metastases may be of different origin and their chemosensitivity may vary according to the histological type and their response in presence of heat.

There still exists a growing need in the art in order to develop new therapeutic strategies for treating cancer, especially metastases systemically. The object of the present invention therefore was to develop such a new strategy. It should be applicable to systemic treatment, and it should lower the dose and/or increase the efficiency of the cancer therapeutical agents to be applied. A further object was to normalize tumor vasculature to increase delivery of systemic therapeutics of tumor, i.e. to reset the tumor vasculature to the functionality of the vasculature of non-tumor tissue.

Thus, it is a preferred objective of the instant invention to provide a more effective, better tolerated treatment for cancer patients leading to enhanced progression-free survival (PFS), QOL and increased median survival.

### Brief description of drawings:

Fig. 1 [for technical information only] shows the results from the rat orthotopic glioblastoma model radiotherapy, cilengitide scheduling experiments. The results are also shown in Table 1.

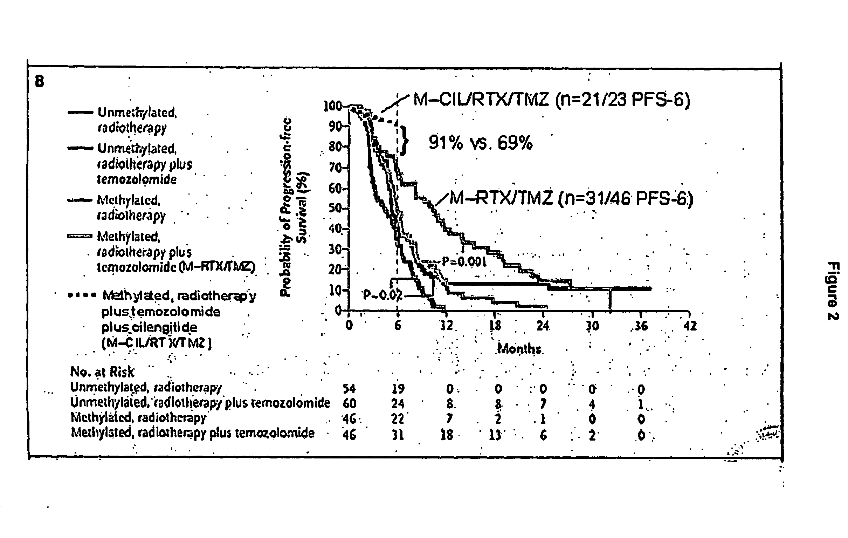

Fig. 2 shows the results of a clinical study in glioblastoma (GBM). The results are also shown in Example 3.

### Summary of the Invention:

Subject of the instant invention is the use of at least one specific integrin ligand, selected from cyclo-(Arg-Gly-Asp-DPhe-NMeVal) and the pharmaceutically acceptable solvates and salts thereof,

for the manufacture of a medicament for the treatment of astrocytoma, wherein the medicament is to be used in combination with temozolomide and radiotherapy.

Subject of the instant invention is Cyclo-(Arg-Gly-Asp-DPhe-NMe-Val) for use for the treatment of astrocytoma in combination with temozolomide and radiotherapy.

It was surprisingly found that the tumor vasculature can be functionally normalized by systemically applied integrin ligands as defined below. Such inhibitors of integrin functions, also referred to as integrin ligands in the context of the present invention, increase the amount of cytotoxics and other cancer cotherapeutic agents entering the tumor. In addition, they have been shown to enhance the numbers of leukocytes entering the tumor following systemic immunocytokines therapy, and may directly increase the amounts of antibodies entering the tumor compartment on anti-tumor antibody therapy, or increase access to anti-tumor vaccines.

Although not known today, the possibility exists that this functional normalization of the tumor vasculature may lead to a higher oxygen concentration in the tumor, and allow oxygen dependent therapies like external beam radiotherapy to become more effective.

The "functional normalizing agent" is defined here empirically as a reagent targeting alpha-v-integrins within the tumor compartment, that increase the levels of systemic tumor therapeutics or of specific bio-indicators of a systemic therapy within the tumor. The increased local therapeutic overcomes tumor resistance mechanisms, and enhances therapeutic index. For example, the systemic therapeutic might be a classical chemotherapeutic reagent, an immunocytokines, a immunotoxin, or a radioimmunotherapy etc. etc.

In this new therapy form, it is a preferred feature that the integrin ligand is administered prior to the further cancer cotherapeutic agent. In this context, according to the present invention, radiation, or, radiotherapy has to be understood as a cancer cotherapeutic agent.

Generally, this prior application takes place 1 to 8 hours (h), preferably 1 to 5 h, and more preferably 1 to 3 h before the application of the further cancer cotherapeutic agent. Even more preferably, this prior application takes place 2 to 8 hours (h), preferably 2 to 6 h, and more preferably 2 to 4 h before the application of the further cancer cotherapeutic agent, such as 1 to 2 h, 2 to 3 h, 3 to 6 h, 2 to 5 h or 3 to 7 h before the application of the further cancer therapeutic agent. With respect to the invention, this prior application or administration is also referred to as "timed administration" or "timed application".

As is shown by the data contained in this application, the effect according to the invention is achieved in non-human animals, especially rats, if this prior application preferably takes place 1 to 8 hours (h), preferably 1 to 5 h, and more preferably 1 to 3 h before the application of the further cancer cotherapeutic agent; and even more preferably this prior application takes place 2 to 8 hours (h), preferably 2 to 6 h, and more preferably 2 to 4 h before the application of the further cancer cotherapeutic agent, such as 1 to 2 h, 2 to 3 h, 3 to 6 h, 2 to 5 h or 3 to 7 h before the application of the further cancer therapeutic agent. With respect to the invention, this prior application or administration is also referred to as "timed administration" or "timed application"

However, the data from experiments with human animals preferably shows that the time of the above/below described and discussed "prior application" can be delayed or multiplied by the factor 1 to 4 and especially 2 to 4. This difference in the response or response time between non-human animals, especially rodents, such as rats, and human animals is known and extensively discussed in the art. While the applicant wishes not to be bound by this theory, he believes that this difference is at least in part caused by the different pharmacokinetic behavior of the different species, which i. a. reflects in different halflives ($t_{1/2}$) in the different kinds of animals. For example, for compounds such as cyclopeptides, the halflives in rats usually are in the range of 10-30 minutes, whereas the halflives in human animals for the same compounds are within 2 to 6 hours and especially 3 to 4 hours.

Accordingly, a subject of this application is a method of treatment and/or a method of manufacture as described above/below, wherein the prior application preferably takes place 1 to 32 hours (h), preferably 2 to 32 h, more preferably 2 to 24 h, even more preferably 4 to 24 h, even more preferably 6 to 20 h and especially 6 to 16 h, before the application of the further cancer cotherapeutic agent; or alternatively preferably this prior application takes place 6 to 32 hours (h), preferably 10 to 24 h, and more preferably 12 to 20 h before the application of the further cancer cotherapeutic agent. With respect to the invention, this prior application or administration is also referred to as "timed administration" or "timed application"

A further subject of this application is a method of treatment and/or a method of manufacture as described above/below, wherein the prior application preferably takes place 18 to 23 h hours (h), preferably 20 to 23 h, more preferably 20 to 22 h before the application of the further cancer cotherapeutic agent; or alternatively preferably this prior application takes place 25 to 32 h hours (h), preferably 25 to 30 h, and more preferably 26 to 30 h before the application of the further cancer cotherapeutic agent. With respect to the invention, this prior application or administration is also referred to as "timed administration" or "timed application"

However, in a more preferred aspect of the instant invention, the timed administration (regardless of whether the patient is a human or nonhuman animal) of the specific integrin ligand takes place 1 to 10 hours (h), preferably 2 to 8 h, more preferably 2 to 6 h, even more preferably 3 to 8 h, even more preferably 3 to 6 h and especially 4 to 8 h prior to the application of the one or more cancer cotherapeutic agents, e.g. 1 to 2 h, 1 to 3 h, 1 to 4 h, 2 to 3 h, 2 to 4 h, 2 to 6 h, 2 to 8 h, 2 to 10 h, 3 to 4 h, 3 to 10 h, 4 to 6 h, 4 to 10 h, 5 to 8 or 5 to 10 h. This is especially preferred if the one or more cancer cotherapeutic agents comprise external beam radiation or consist of external beam radiation. With respect to the invention, this prior application or administration is also referred to as "timed administration" or "timed application".

With respect to said timed administration or timed application (of the specific integrin ligand), the hours given for said prior administration or application preferably refer to the beginning or start of the respective administration or application. Accordingly, for example, an administration of the specific integrin ligand starting three hours before the application of the respective cancer cotherapeutic agent is to be regarded as a timed administration or timed application 3 h prior to the application of the one or more cancer cotherapeutic agents according to the invention, even if the specific integrin ligand is administered by i. v. Infusion that takes an hour or two hours to be completed. This definition of prior application/prior administration is in perfect concordance with the understanding of the ones skilled in the art.

If the at least one specific integrin ligand is administered to the patient in a timed administration as described herein, it is preferably timed with respect to the one or more cancer cotherapeutic agents it is combined with. With respect to the timed administration of the specific integrin ligand in combination with two or more cancer cotherapeutic agents, it is preferably timed with respect to the two or more cancer cotherapeutic agents, more preferably timed with respect to at least one of the cancer cotherapeutic agents. If the one or more cancer cotherapeutic agents comprise radiotherapy, especially radiotherapy as described herein, the timed administration preferably refers at least to the radiotherapy.

Especially preferably, the timed administration of the specific integrin ligand refers to radiotherapy as the time-relevant cancer cotherapeutic. Accordingly, in the timed administration, the prior administration of the specific integrin ligand preferably refers to a time prior to the administration of radiotherapy. However, in many cases, it can be advantageous also to administer the one or more further cancer cotherapeutic agents other than radiotherapy within the time window given by the timed administration of the specific integrin ligand and the administration or delivery of the radiotherapy.

Regarding the timed administration of at least one specific integrin ligand, selected from the group consisting of cyclo-(Arg-Gly-Asp-DPhe-NMe-Val), the pharmaceutically acceptable solvates and/or salts thereof, and the cancer cotherapeutic agents temozolomide and radiotherapy, the timed administration of the at least one specific integrin ligand preferably refers to the administration or delivery of the radiotherapy, and more preferably to the administration or delivery radiotherapy and the administration of the temozolomide.

More preferably, the timed administration of the specific integrin ligand refers to the administration of the specific integrin ligand and the radiotherapy, and the additional cancer cotherapeutic agent temozolomide, is preferably administered after the administration of the specific integrin ligand, such as 1 to 2 or 1 to 3 hours after the administration of this specific integrin ligand, but preferably before the administration or delivery of the radiotherapy, preferably at least within one hour before the administration or delivery of the radiotherapy, and more preferably at least 1 hour before radiotherapy, for example 1 to 2 or 1 to 3 h prior to the administration or delivery of the radiotherapy.

If two or more specific integrin ligands are administered in a timed administration as described herein, the timed and administration preferably refers at least to one of the specific integrin ligands and more preferably to the two or more specific integrin ligands to be administered in the timed administration as described herein.

It should be understood that the administration of any combination for use according to the present invention is accompanied by radiation therapy, wherein radiation treatment can preferably be done after the administration of the integrin ligand. The administration of the different agents of the combination therapy according to the invention can optionally also be achieved substantially concurrently or sequentially.

It is known that tumors elicit alternative routes for their development and growth. If one route is blocked they often have the capability to switch to another route by expressing and using other receptors and signaling pathways. Therefore, the pharmaceutical combinations for use according to the present invention may block several of such possible development strategies of the tumor and provide consequently various therapeutic benefits. The combinations for use according to the present invention are used in treating and preventing astrocytoma.

Preferably, the different combined agents for use according to the present invention are administered at a low dose, that is, at a dose lower than has been conventionally used in clinical situations. A benefit of lowering the dose of the compounds, compositions, agents and therapies administered to an individual includes a decrease in the incidence of adverse effects associated with higher dosages. For example, by the lowering the dosage of an agent described above and below, a reduction in the frequency and the severity of nausea and vomiting will result when compared to that observed at higher dosages. By lowering the incidence of adverse effects, an improvement in the quality of life of a cancer patient is expected. Further benefits of lowering the incidence of adverse effects include an improvement in patient compliance, a reduction in the number of hospitalizations needed for the treatment of adverse effects, and a reduction in the administration of analgesic agents needed to treat pain associated with the adverse effects. Alternatively, the methods and combination can also maximize the therapeutic effect at higher doses.

The combinations within the pharmaceutical treatment for use according to the invention show an astonishing synergetic effect. In administering the combination of drugs real tumor shrinking and disintegration could be observed during clinical studies while no significant adverse drug reactions were detectable.

The pharmaceutical combinations for use according to the present invention provide various benefits. The combinations for use according to the present invention are used in treating and preventing astrocytoma. Preferably, the different combined agents for use according to the present invention are to be administered in combination at a low dose, that is, at a dose lower than has been conventionally used in clinical situations. A benefit of lowering the dose of the compounds, compositions, agents and therapies administered to a mammal includes a decrease in the incidence of adverse effects associated with higher dosages. For example, by the lowering the dosage of a chemotherapeutic agent such as methotrexate, doxorubicin, gemcitabine, docetaxel, paclitaxel, bleomycin, cisplatin and/or Melphalan, a reduction in the frequency and the severity of nausea and vomiting will result when compared to that observed at higher dosages. Similar benefits are contemplated for the compounds, compositions, agents and therapies in combination with the integrin antagonist for use according to the present invention. By lowering the incidence of adverse effects, an improvement in the quality of life of a cancer patient is contemplated. Further benefits of lowering the incidence of adverse effects include an improvement in patient compliance, a reduction in the number of hospitalizations needed for the treatment of adverse effects, and a reduction in the administration of analgesic agents needed to treat pain associated with the adverse effects.

Alternatively, the methods and combination can also maximize the therapeutic effect at higher doses.

## Detailed Description of the Invention

If not otherwise pointed out, the terms and phrases used in this invention preferably have the meanings and definitions as given below. Moreover, these definitions and meanings describe the invention in more detail, preferred embodiments included.

If not otherwise pointed out, the reference to a compound for use according to the invention preferably includes the reference to the pharmaceutically acceptable solvates and salts thereof. If not otherwise pointed out, the reference to the integrin ligands, integrin antagonists, integrin agonists, as well as the reference to the cancer-cotherapeutic agents that are compounds, preferably includes the pharmaceutically acceptable solvates and salts thereof. Even more preferably, the reference to the integrin ligand cyclo-(Arg-Gly-Asp-DPhe-NMeVal) also includes the pharmaceutically acceptable solvates and salts thereof, and especially preferably the pharmaceutically acceptable salts thereof, if not indicated otherwise.

By **"combination therapy"** is preferably meant a combination of at least two distinct therapy forms so combined as to form a single therapeutical concept in a timely controlled, consecutive manner.

In a preferred embodiment of the present invention this means the combination of the specific integrin ligand with the further cotherapeutic agent temololomide. It is important to note that **"combination therapy"** preferably does not mean a distinct and/or single pharmaceutical composition or medicament. By way of contrast, in a preferred embodiment of the present invention the integrin ligand and the further cotherapeutic agent temozolomide are provided in discrete containers, packages, medicaments, formulations or equivalents. Equally, the combination of integrin ligand therapy with radiation therapy preferably lies within the meaning of **"combination therapy"** of the present invention.

**"Therapy forms"** preferably are any means, uses and/or formulations for treating cancer known in the art. By the term "distinct therapy forms" therefore it is meant that two different means, uses and/or formulations for treating astrocytoma are combined. In the context of the present invention it is preferred that the first to be applied **therapy form** has anti-integrin activity (synonym: integrin ligand), and is applied prior to the second **therapy form**, preferably following the schedule as detailed above.

The term "**composition comprising radiotherapy**" preferably simply means that subsequent to the integrin ligand radiotherapy is applied. Therefore, the term **"composition comprising radiotherapy"** in the context of the present invention preferably does not apply to a pharmaceutical composition as such, but to a pharmaceutical composition to be used in combination with radiotherapy.

With **"cancer-cotherapeutic agent"** or **"cotherapeutic agent"** preferably a cytotoxic, chemotherapeutical or immunotoxic agent is meant. Equally preferred is radiotherapy.

A **"receptor"** or **"receptor molecule"** is preferably a soluble or membrane bound or membrane associated protein or glycoprotein comprising one or more domains to which a ligand binds to form a receptor-ligand complex. By binding the ligand, which may be an agonist or an antagonist the receptor is activated or inactivated and may initiate or block pathway signaling.

By **"ligand"** or **"receptor ligand"** is preferably meant a natural or synthetic compound which binds a receptor molecule to form a receptor-ligand complex. The term ligand includes agonists, antagonists, and compounds with partial agonist/antagonist activity.

An **"agonist"** or **"receptor agonist"** is preferably a natural or synthetic compound which binds the receptor to form a receptor-agonist complex by activating said receptor and receptor-agonist complex, respectively, initiating a pathway signaling and further biological processes.

By **"antagonist"** or **"receptor antagonists"** is preferably meant a natural or synthetic compound that has a biological effect opposite to that of an agonist. An antagonist binds the receptor and blocks the action of a receptor agonist by competing with the agonist for receptor. An antagonist is defined by its ability to block the actions of an agonist. A receptor antagonist may be also an antibody or an immunotherapeutically effective fragment thereof. Preferred antagonists according to the present invention are cited and discussed below.

The term **"integrin antagonists / inhibitors"** or **"integrin receptor antagonists / inhibitors"** preferably refers to a natural or synthetic molecule, preferably a synthetic molecule, that blocks and inhibit an integrin receptor. In some cases, the term includes antagonists directed to the ligands of said integrin receptors (such as for $\alpha_v\beta_3$: vitronectin, fibrin, fibrinogen, von Willebrand's factor, thrombospondin, laminin; for $\alpha_v\beta_5$: vitronectin; for $\alpha_v\beta_1$: fibronectin and vitronectin; for $\alpha_v\beta_6$: fibronectin). Integrin (receptor) antagonists may be natural or synthetic peptides, non-peptides, peptidomimetica, immunoglobulins, such as antibodies or functional fragments thereof, or immunoconjugates (fusion proteins). Integrin inhibitors are directed to receptor of $\alpha_v$ integrins (e.g. $\alpha_v\beta_3$, $\alpha_v\beta_5$, $\alpha_v\beta_6$ and sub-classes). Integrin inhibitors are $\alpha_v$ antagonists, and in particular $\alpha_v\beta_3$ antagonists. $\alpha_v$ antagonists are RGD peptides, peptidomimetic (non-peptide) antagonists and anti-integrin receptor antibodies such as antibodies blocking $\alpha_v$ receptors.

Exemplary, non-immunological $\alpha_v\beta_3$ antagonists are described in the teachings of US 5,753,230 and US 5,766,591. Antagonists are linear and cyclic RGD-containing peptides. Cyclic peptides are, as a rule, more stable and elicit an enhanced serum half-life. The integrin antagonist for use according to the invention is, however, cyclo-(Arg-Gly-Asp-DPhe-NMeVal) (EMD 121974, Cilengitide®, Merck KGaA, Germany; EP 0770 622) which is efficacious in blocking the integrin receptors $\alpha_v\beta_3$, $\alpha_v\beta_1$, $\alpha_v\beta_6$, $\alpha_v\beta_8$, $\alpha_{IIb}\beta_3$, and preferably especially efficacious with respect to integrin receptors $\alpha_v\beta_3$ and/or $\alpha_v\beta_5$. As is clear to the ones skilled in the art, the cyclo-(Arg-Gly-Asp-DPhe-NMeVal) can be also applied in the context of the instant invention in the form of A physiologically acceptable solvate and/or a salt thereof. The same preferably also applies to all other compounds or active ingredients to be used in the context of the present invention.

Peptidyl as well as peptidomimetic (non-peptide) antagonists of the $\alpha_v\beta_3$ / $\alpha_v\beta_5$ / $\alpha_v\beta_6$ integrin receptor have been described both in the scientific and patent literature. For example, reference is made to Hoekstra and Poulter, 1998, Curr. Med. Chem. 5, 195; WO 95/32710; WO 95/37655; WO 97/01540; WO 97/37655; WO 97/45137; WO 97/41844; WO 98/08840; WO 98/18460; WO 98/18461; WO 98/25892; WO 98/31359; WO 98/30542; WO 99/15506; WO 99/15507; WO 99/31061; WO 00/06169; EP 0853 084; EP 0854 140; EP 0854 145; US 5,780,426; and US 6,048,861. Patents that disclose benzazepine, as well as related benzodiazepine and benzocycloheptene $\alpha_v\beta_3$ integrin receptor antagonists include WO 96/00574, WO 96/00730, WO 96/06087, WO 96/26190, WO 97/24119, WO 97124122, WO 97/24124, WO 98/15278, WO 99/05107, WO 99/06049, WO 99/15170, WO 99/15178, WO 97/34865, WO 97/01540, WO 98/30542, WO 99/11626, and WO 99/15508. Other integrin receptor antagonists featuring backbone conformational ring constraints have been described in WO 98/08840; WO 99/30709; WO 99/30713; WO 99/31099; WO 00/09503; US 5,919,792; US 5,925,655; US 5,981,546; and US 6,017,926. In US 6,048,861 and WO 00/72801 a series of nonanoic acid derivatives which are potent $\alpha_v\beta_3$ integrin receptor antagonists were disclosed. Other chemical small molecule integrin antagonists (mostly vitronectin antagonists) are described in WO 00/38665. Other $\alpha_v\beta_3$ receptor antagonists have been shown to be effective in inhibiting angiogenesis. For example, synthetic receptor antagonists such as (S)-10,11-Dihydro-3-[3-(pyridin-2-ylamino)-1-propyloxy]-5H-dibenzo[ a,d]cycloheptene-10-acetic acid (known as SB-265123) have been tested in a variety of mammalian model systems. (Keenan et al., 1998, Bioorg. Med. Chem. Lett. 8(22), 3171; Ward et al., 1999, Drug Metab. Dispos. 27(11), 1232). Assays for the identification of integrin antagonists suitable for use as an antagonist are described, e.g. by Smith et al., 1990, J. Biol. Chem. 265, 12267, and in the referenced patent literature. Anti-integrin receptor antibodies are also well known. Suitable anti-integrin (e.g. $\alpha_v\beta_3$, $\alpha_v\beta_5$, $\alpha_v\beta_6$) monoclonal antibodies can be modified to encompass antigen binding fragments thereof, including F(ab)$_2$, Fab, and engineered Fv or single-chain antibody. One monoclonal antibody directed against integrin receptor $\alpha_v\beta_3$ is identified as LM609 (Brooks et al., 1994, Cell 79, 1157; ATCC HB 9537). A potent specific anti-$\alpha_v\beta_5$ antibody, P1F6, is disclosed in WO 97/45447 . A further $\alpha_v\beta_6$ selective antibody is MAb 14D9.F8 (WO 99/37683, DSM ACC2331, Merck KGaA, Germany) which is selectively directed to the $\alpha_v$- chain of integrin receptors. Another anti-integrin antibody is the commercialized Vitraxin ®.

The term "**antibody**" or "**immunoglobulin**" herein is preferably used in the broadest sense and specifically covers intact monoclonal antibodies, polyclonal antibodies, multispecific antibodies (e.g. bispecific antibodies) formed from at least two intact antibodies, and antibody fragments, so long as they exhibit the desired biological activity. The term generally includes heteroantibodies which are composed of two or more antibodies or fragments thereof of different binding specificity which are linked together.

Depending on the amino acid sequence of their constant regions, intact antibodies can be assigned to different "**antibody (immunoglobulin) classes**". There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called $\alpha$, $\delta$, $\varepsilon$, $\gamma$ and $\mu$ respectively. Preferred major class for antibodies according to the invention is IgG, in more detail IgG1 and IgG2.

Antibodies are usually glycoproteins having a molecular weight of about 150,000, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intra-chain disulfide bridges. Each heavy chain has at one end a variable domain (VH) followed by a number of constant domains. The variable regions comprise hypervariable regions or "**CDR**" regions, which contain the antigen binding site and are responsible for the specificity of the antibody, and the "**FR**" regions, which are important with respect to the affinity / avidity of the antibody. The hypervariable region generally comprises amino acid residues from a "complementarity determining region" or "CDR" (e.g. residues 24-34 (L1), 50-56 (L2) and 89-97 (L3) in the light chain variable domain and 31-35 (H1), 50-65 (H2) and 95-102 (H3) in the heavy chain variable domain; and/or those residues from a "hypervariable loop" (e.g. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)).The "**FR**" residues (frame work region) are those variable domain residues other than the hypervariable region residues as herein defined. Each light chain has a variable domain at one end (VL) and a constant domain at its other end. The constant domain of the light chain is aligned with the first constant domain of the heavy chain, and the light-chain variable domain is aligned with the variable domain of the heavy chain.

Particular amino acid residues are believed to form an interface between the light chain and heavy chain variable domains. The "light chains" of antibodies from any vertebrate species can be assigned to one of two clearly distinct types, called kappa ($\kappa$) and lambda ($\lambda$), based on the amino acid sequences of their constant domains.

The term "**monoclonal antibody**" as used herein preferably refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. Methods for making monoclonal antibodies include the hybridoma method described by Kohler and Milstein (1975, Nature 256, 495) and in "Monoclonal Antibody Technology, The Production and Characterization of Rodent and Human Hybridomas" (1985, Burdon et al., Eds, Laboratory Techniques in Biochemistry and Molecular Biology, Volume 13, Elsevier Science Publishers, Amsterdam), or may be made by well known recombinant DNA methods (see, e.g., US 4,816,567). Monoclonal antibodies may also be isolated from phage antibody libraries using the techniques described in Clackson et al., Nature, 352:624-628 (1991) and Marks et al., J. Mol. Biol., 222:58, 1-597(1991), for example.

The term "**chimeric antibody**" preferably means antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (e.g.: US 4,816,567; Morrison et al., Proc. Nat. Acad. Sci., USA, 81:6851-6855 (1984)). Methods for making chimeric and humanized antibodies are also known in the art. For example, methods for making chimeric antibodies include those described in patents by Boss (Celltech) and by Cabilly (Genentech) (US 4,816,397; US 4,816,567).

"**Humanized antibodies**" preferably are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (US 5,225,539) and Boss (Celltech, US 4,816,397).

"**Antibody fragments**" preferably comprise a portion of an intact antibody, preferably comprising the antigen-binding or variable region thereof. Examples of antibody fragments include Fab, Fab', $F(ab')_2$, Fv and Fc fragments, diabodies, linear antibodies, single-chain antibody molecules; and multispecific antibodies formed from antibody fragment(s). An "intact" antibody is one which comprises an antigen-binding variable region as well as a light chain constant domain (CL) and heavy chain constant domains, CH1, CH2 and CH3. Preferably, the intact antibody has one or more effector functions. Papain digestion of antibodies produces two identical antigen-binding fragments, called "**Fab**" fragments, each comprising a single antigen-binding site and a CL and a CH1 region, and a residual "Fc" fragment, whose name reflects its ability to crystallize readily. The "**Fc**" region of the antibodies comprises, as a rule, a CH2, CH3 and the hinge region of an IgG1 or IgG2 antibody major class. The hinge region is a group of about 15 amino acid residues which combine the CH1 region with the CH2-CH3 region. Pepsin treatment yields an "**F(ab')2**" fragment that has two antigen-binding sites and is still capable of cross-linking antigen. "Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions (CDRs) of each variable domain interact to define an antigen-binding site on the surface of the VH - VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site. The **Fab** fragment also contains the constant domain of the light chain and the first constant domain (CH1) of the heavy chain. "**Fab'**" fragments differ from Fab fragments by the addition of a few residues at the carboxy terminus of the heavy chain CH1 domain including one or more cysteines from the antibody hinge region. F(ab')2 antibody fragments originally were produced as pairs of Fab' fragments which have hinge cysteines between them. Other chemical couplings of antibody fragments are also known (see e.g. Hermanson, Bioconjugate Techniques, Academic Press, 1996; US 4,342,566). "**Single-chain Fv**" or "**scFv**" antibody fragments preferably comprise the V, and V, domains of antibody, wherein these domains are present in a Single polypeptide chain. Preferably, the Fv polypeptide further comprises a polypeptide linker between the VH and VL domains which enables the scFv to form the desired structure for antigen binding. Single-chain FV antibodies are known, for example, from Plückthun (The Pharmacology of Monoclonal Antibodies, Vol. 113, Rosenburg and Moore eds., Springer-Verlag, New York, pp. 269-315 (1994)), WO.93/16185; US 5,571,894; US 5,587,458; Huston et al. (1988, Proc. Natl. Acad. Sci. 85, 5879) or Skerra and Plueckthun (1988, Science 240, 1038).

"**Bispecific antibodies**" preferably are single, divalent antibodies (or immunotherapeutically effective fragments thereof) which have two differently specific antigen binding sites. For example the first antigen binding site is directed to an angiogenesis receptor (e.g. integrin or VEGF receptor), whereas the second antigen binding site is directed to an ErbB receptor (e.g. EGFR or Her 2). Bispecific antibodies can be produced by chemical techniques (see e.g., Kranz et al. (1981) Proc. Natl. Acad. Sci. USA 78, 5807), by "polydoma" techniques (See US 4,474,893) or by recombinant DNA techniques, which all are known per se. Further methods are described in WO 91/00360, WO 92/05793 and WO 96/04305. Bispecific antibodies can also be prepared from single chain antibodies (see e.g., Huston et al. (1988) Proc. Natl. Acad. Sci. 85, 5879; Skerra and Plueckthun (1988) Science 240, 1038). These are analogues of antibody variable regions produced as a single polypeptide chain. To form the bispecific binding agent, the single chain antibodies may be coupled together chemically or by genetic engineering methods known in the art. It is also possible to produce bispecific antibodies according to this invention by using leucine zipper sequences. The sequences employed are derived from the leucine zipper regions of the transcription factors Fos and Jun (Landschulz et al., 1988, Science 240,1759; for review, see Maniatis and Abel, 1989, Nature 341, 24). Leucine zippers are specific amino acid sequences about 20-40 residues long with leucine typically occurring at every seventh residue. Such zipper sequences form amphipathic $\alpha$-helices, with the leucine residues lined up on the hydrophobic side for dimer formation. Peptides corresponding to the leucine zippers of the Fos and Jun proteins form heterodimers preferentially (O'Shea et al., 1989, Science 245, 646). Zipper containing bispecific antibodies and methods for making them are also disclosed in WO 92/10209 and WO 93/11162. A bispecific antibody according the invention may be an antibody, directed to VEGF receptor and $\alpha v\beta 3$ receptor as discussed above with respect to the antibodies having single specificity.

"**Heteroantibodies**" preferably are two or more antibodies or antibody-binding fragments which are linked together, each of them having a different binding specificity. Heteroantibodies can be prepared by conjugating together two or more antibodies or antibody fragments. Preferred heteroantibodies are comprised of cross-linked Fab/Fab' fragments. A variety of coupling or crosslinking agents can be used to conjugate the antibodies. Examples are protein A, carboimide, N-succinimidyl-S-acetyl-thioacetate (SATA) and N-succinimidyl-3-(2-pyridyldithio) propionate (SPDP) (see e.g., Karpovsky et al. (1984) J. EXP. Med. 160,1686; Liu et a. (1985) Proc. Natl. Acad. Sci. USA 82, 8648). Other methods include those described by Paulus, Behring Inst. Mitt., No. 78, 118 (1985); Brennan et a. (1985) Science 30 Method:81 or Glennie et al. (1987) J. Immunol. 139, 2367. Another method uses o-phenylenedimaleimide (oPDM) for coupling three Fab' fragments (WO 91/03493). Multispecific antibodies are in context of this invention also suitable and can be prepared, for example according to the teaching of WO 94/13804 and WO 98/50431.

The term "**fusion protein**" preferably refers to a natural or synthetic molecule consisting of one ore more proteins or peptides or fragments thereof having different specificity which are fused together optionally by a linker molecule. As specific embodiment the term includes fusion constructs, wherein at least one protein or peptide is a immunoglobulin or antibody, respectively or parts thereof ("immunoconjugates").

The term "**immunoconjugate**" preferably refers to an antibody or immunoglobulin respectively, or a immunologically effective fragment thereof, which is fused by covalent linkage to a non-immunologically effective molecule. Preferably this fusion partner is a peptide or a protein, which may be glycosylated. Said non-antibody molecule can be linked to the C-terminal of the constant heavy chains of the antibody or to the N-terminals of the variable light and/or heavy chains. The fusion partners can be linked via a linker molecule, which is, as a rule, a 3 - 15 amino acid residues containing peptide. Immunoconjugates according to the invention consist of an immunoglobulin or immunotherapeutically effective fragment thereof, directed to a receptor tyrosine kinase, preferably an ErbB (ErbB1/ErbB2) receptor and an integrin antagonistic peptide, or an angiogenic receptor, preferably an integrin or VEGF receptor and TNF$\alpha$ or a fusion protein consisting essentially of TNF$\alpha$ and IFN$\gamma$ or another suitable cytokine, which is linked with its N-terminal to the C-terminal of said immunoglobulin, preferably the Fc portion thereof. The term includes also corresponding fusion constructs comprising bi- or multi-specific immunoglobulins (antibodies) or fragments thereof.

The term "**cytokine**" is preferably a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; mouse gonadotropin-associated peptide; inhibin; activin; vascular endothelial growth factor (VEGF); integrin; thrombopoietin (TPO); nerve growth factors such as NGF$\beta$; platelet-growth factor; transforming growth factors (TGFs) such as TGF$\alpha$ and TGF$\beta$; erythropoietin (EPO); interferons such as IFN$\alpha$, IFN$\beta$, and IFN$\gamma$; colony stimulating factors such as M-CSF, GM-CSF and G-CSF; interleukins such as IL-1, IL-1a, IL-2, IL-3, IL-4, IL-5, IL-6, IL-7, IL-8, IL-9, IL-10, IL-11, IL-12; and TNF$\alpha$ or TNF$\beta$. Preferred cytokines according to the invention are interferons and TNF$\alpha$.

The term "**cytotoxic agent**" as used herein preferably refers to a substance that inhibits or prevents the function of cells and/or causes destruction of cells. The term is preferably intended to include radioactive isotopes, chemotherapeutic agents, and toxins such as enzymatically active toxins of bacterial, fungal, plant or animal origin, or fragments thereof. The term may include also members of the cytokine family, preferably IFN$\gamma$ as well as anti-neoplastic agents having also cytotoxic activity.

The term "**chemotherapeutic agent**", "**chemotherapeutical agent**" or "**anti-neoplastic agent**" is regarded according to the understanding of this invention preferably as a member of the class of "cytotoxic agents", as specified above, and includes chemical agents that exert anti-neoplastic effects, i.e., prevent the development, maturation, or spread of neoplastic cells, directly on the tumor cell, e.g., by cytostatic or cytotoxic effects, and not indirectly through mechanisms such as biological response modification. Suitable chemotherapeutic agents according to the invention are preferably natural or synthetic chemical compounds, but biological molecules, such as proteins, polypeptides etc. are not expressively excluded. There are large numbers of anti-neoplastic agents available in commercial use, in clinical evaluation and in pre-clinical development, which could be included in the present invention for treatment of tumors / neoplasia by combination therapy with TNF$\alpha$ and the anti-angiogenic agents as cited above, optionally with other agents such as EGF receptor antagonists. It should be pointed out that the chemotherapeutic agents can be administered optionally together with above-said drug combination. Examples of chemotherapeutic or agents include alkylating agents, for example, nitrogen mustards, ethyleneimine compounds, alkyl sulphonates and other compounds with an alkylating action such as nitrosoureas, cisplatin and dacarbazine; antimetabolites, for example, folic acid, purine or pyrimidine antagonists; mitotic inhibitors, for example, vinca alkaloids and derivatives of podophyllotoxin; cytotoxic antibiotics and camptothecin derivatives. Preferred chemotherapeutic agents or chemotherapy include amifostine (ethyol), cisplatin, dacarbazine (DTIC), dactinomycin, mechlorethamine (nitrogen mustard), streptozocin, cyclophosphamide, carrnustine (BCNU), lomustine (CCNU), doxorubicin (adriamycin), doxorubicin lipo (doxil), gemcitabine (gemzar), daunorubicin, daunorubicin lipo (daunoxome), procarbazine, mitomycin, cytarabine, etoposide, methotrexate, 5-fluorouracil (5-FU), vinblastine, vincristine, bleomycin, paclitaxel (taxol), docetaxel (taxotere), aldesleukin, asparaginase, busulfan, carboplatin, cladribine, camptothecin, CPT-11, 10-hydroxy-7-ethyl-camptothecin (SN38), dacarbazine, floxuridine, fludarabine, hydroxyurea, ifosfamide, idarubicin, mesna, interferon alpha, interferon beta, irinotecan, mitoxantrone, topotecan, leuprolide, megestrol, melphalan, mercaptopurine, plicamycin, mitotane, pegaspargase, pentostatin, pipobroman, plicamycin, streptozocin, tamoxifen, teniposide, testolactone, thioguanine, thiotepa, uracil mustard, vinorelbine, chlorambucil and combinations thereof.

Preferred chemotherapeutic agents according to the invention include cisplatin, gemcitabine, temozolomide, doxorubicin, paclitaxel (taxol) and bleomycin.

The term "**immunotoxic**" preferably refers to an agent which combines the specifity of a immunomolecule .e.g. an antibody or a functional equivalent thereof with a toxic moiety, e.g. a cytotoxic function as defined above.

Further examples of cancer cotherapeutic agents and preferably of chemotherapeutical agents, cytotoxic agents, immunomodulating agents and/or immunotoxic agents preferably include antibodies against one or more target, preferably selected from the group consisting of HER, HER2, PDGF, PDGFR, EGF, EGFR, VEGF, VEGFR and/or VEGFR2, wherein said antibodies are preferably selected from Herceptin, Bevacizumab (rhuMAb-VEGF, Avastin®), Cetuximab (Erbitux®) and Nimotuzumab, and preferably small molecules or NCEs against one or more of said targets, preferably selected from the group consisting of Sorafenib (Nexavar®), Sunitinib (Sutent®) and ZD6474 (ZACTIMAT™).

In a preferred aspect of the instant invention, the chemotherapeutical agents, cytotoxic agents, immunomodulating agents and/or immunotoxic agents are selected from one or more of the following groups:

a) alkylating agents,
b) antibiotics,
c) antimetabolites,
d) biologicals and immunomodulators,
e) hormones and antagonists thereof,
f) mustard gas derivatives,
g) alkaloids,
h) protein kinase inhibitors.

In a more preferred aspect of the instant invention, the chemotherapeutical agents, cytotoxic agents, immunomodulating agents and/or immunotoxic agents are selected from one or more of the following groups:

a) alkylating agents, selected from busulfan, melphalan, carboplatin, cisplatin, cyclophosphamide, dacarbazine, carmustine (BCNU), nimustin (ACNU), lomustine (CCNU), ifosfamide, temozolomide and altretamine,
b) antibiotics, selected from leomycin, doxorubicin, adriamycin, idarubicin, epirubicin and plicamycin,
c) antimetabolites, selected from sulfonamides, folic acid antagonists, gemcitabine, 5-fluorouracil (5-FU), leucovorine, leucovorine with 5-FU, 5-FU with calcium folinate, and leucovorin, capecitabine, mercaptopurine, cladribine, pentostatine, methotrexate, raltitrexed, pemetrexed, thioguanine, camptothecin derivates (topotecan, irinotecan)
d) biologicals and immunomodulators, selected from interferon a2A, interleukin 2 and levamisole,
e) hormones and antagonists thereof, selected from flutamide, goserelin, mitotane and tamoxifen,
f) mustard gas derivatives, selected from melphalan, carmustine and nitrogen mustard,

EP 1 973 569 B1

g) alkaloids, selected from taxanes, docetaxel, paclitaxel, etoposide, vincristine, vinblastine and vinorelbine.

Dosings and preferably standard administration schedules for the above given cancer cotherapapeutic agents are known in the art.

The terms **"cancer" and "tumor"** preferably refer to or describe the physiological condition in mammals that is typically characterized by unregulated cell growth, such as tumors of the breast, heart, lung, small intestine, colon, spleen, kidney, bladder, head and neck, ovary, prostate, brain, pancreas, skin, bone, bone marrow, blood, thymus, uterus, testicles, cervix, and liver, or more specifically adenoma, angio-sarcoma, astrocytoma, epithelial carcinoma, germinoma, glioblastoma, glioma, hamartoma, hemangioendothelioma, hemangiosarcoma, hematoma, hepato-blastoma, leukemia, lymphoma, medulloblastoma, melanoma, neuroblastoma, osteosarcoma, retinoblastoma, rhabdomyosarcoma, sarcoma and teratoma.

In detail, the tumor/cancer can be acral lentiginous melanoma, actinic keratoses, adenocarcinoma, adenoid cycstic carcinoma, adenomas, adenosarcoma, adenosquamous carcinoma, astrocytic tumors, bartholin gland carcinoma, basal cell carcinoma, bronchial gland carcinomas, capillary, carcinoids, carcinoma, carcinosarcoma, cavernous, cholangiocarcinoma, chondosarcoma, choriod plexus papilloma/carcinoma, clear cell carcinoma, cystadenoma, endodermal sinus tumor, endometrial hyperplasia, endometrial stromal sarcoma, endometrioid adenocarcinoma, ependymal, epitheloid, Ewing's sarcoma, fibrolamellar, focal nodular hyperplasia, gastrinoma, germ cell tumors, glioblastoma, glucagonoma, hemangiblastomas, hemangioendothelioma, hemangiomas, hepatic adenoma, hepatic adenomatosis, hepatocellular carcinoma, insulinoma, intaepithelial neoplasia, interepithelial squamous cell neoplasia, invasive squamous cell carcinoma, large cell carcinoma, leiomyosarcoma, lentigo maligna melanomas, malignant melanoma, malignant mesothelial tumors, medulloblastoma, medulloepithelioma, melanoma, meningeal, mesothelial, metastatic carcinoma, mucoepidermoid carcinoma, neuroblastoma, neuroepithelial adenocarcinoma nodular melanoma, oat cell carcinoma, oligodendroglial, osteosarcoma, pancreatic polypeptide, papillary serous adeno-carcinoma, pineal cell, pituitary tumors, plasmacytoma, pseudo-sarcoma, pulmonary blastoma, renal cell carcinoma, retinoblastoma, rhabdomyo-sarcoma, sarcoma, serous carcinoma, small cell carcinoma, soft tissue carcinomas, somatostatin-secreting tumor, squamous carcinoma, squamous cell carcinoma, submesothelial, superficial spreading melanoma, undifferentiated carcinoma, uveal melanoma, verrucous carcinoma, vipoma, well differentiated carcinoma, and Wilm's tumor.

The tumor/cancer is selected from the group consisting of astrocytoma, preferably astrocytoma grade II, III or IV, glioblastoma, and especially preferably glioblastoma multiforme (GBM).

The **"pharmaceutical compositions"** can comprise agents that reduce or avoid side effects associated with the combination therapy of the present invention ("adjunctive therapy"), including, but not limited to, those agents, for example, that reduce the toxic effect of anticancer drugs, e.g., bone resorption inhibitors, cardioprotective agents. Said adjunctive agents prevent or reduce the incidence of nausea and vomiting associated with chemotherapy, radiotherapy or operation, or reduce the incidence of infection associated with the administration of myelosuppressive anticancer drugs. Adjunctive agents are well known in the art. The immunotherapeutic agents according to the invention can additionally administered with adjuvants like BCG and immune system stimulators. Furthermore, the compositions may include immunotherapeutic agents or chemotherapeutic agents which contain cytotoxic effective radio labeled isotopes, or other cytotoxic agents, such as a cytotoxic peptides (e.g. cytokines) or cytotoxic drugs and the like.

The term " **pharmaceutical kit**" for treating tumors or tumor metastases refers to a package and, as a rule, instructions for using the reagents in methods to treat tumors and tumor metastases. A reagent in a kit of this invention is typically formulated as a therapeutic composition as described herein, and therefore can be in any of a variety of forms suitable for distribution in a kit. Such forms can include a liquid, powder, tablet, suspension and the like formulation for providing the antagonist and/or the fusion protein of the present invention. The reagents may be provided in separate containers suitable for administration separately according to the present methods, or alternatively may be provided combined in a composition in a single container in the package. The package may contain an amount sufficient for one or more dosages of reagents according to the treatment methods described herein. A kit of this invention also contains "instruction for use" of the materials contained in the package.

As used herein the terms **"pharmaceutically acceptable"** and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like. The preparation of a pharmacological composition that contains active ingredients dissolved or dispersed therein is well understood in the art and need not be limited based on formulation. Typically, such compositions are prepared as injectables either as liquid solutions or suspensions, however, solid forms suitable for solution, or suspensions, in liquid prior to use can also be prepared. The preparation can also be emulsified. The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents., pH buffering agents and the like which enhance the effectiveness of the active ingredient. The therapeutic composition of the present invention can include pharmaceutically acceptable salts of the components therein. Pharmaceutically acceptable salts include the acid addition salts (formed with the free amino groups of the polypeptide) that are formed with inorganic acids such as for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. Particularly preferred is the HCl salt when used in the preparation of cyclic polypeptide αv antagonists. Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes. Liquid compositions can also contain liquid phases in addition to and to the exclusion of water. Exemplary of such additional liquid phases are glycerin vegetable oils such as cottonseed oil, and water-oil emulsions. Typically, a therapeutically effective amount of an immunotherapeutic agent in the form of a, for example, antibody or antibody fragment or antibody conjugate is an amount such that when administered in physiologically tolerable composition is sufficient to achieve a plasma concentration of from about 0.01 microgram (μg) per milliliter (ml) to about 100 μg/ml, preferably from about 1 μg/ml to about 5 μg/ml and usually about 5 μg/ml. Stated differently the dosage can vary from about 0.1 mg/kg to about 300 mg/kg, preferably from about 0.2 mg/kg to about 200 mg/kg, most preferably from about 0.5 mg/kg to about 20 mg/kg, in one or more dose administrations daily for one or several days. Where the immunotherapeutic agent is in the form of a fragment of a monoclonal antibody or a conjugate, the amount can readily be adjusted based on the mass of the fragment / conjugate relative to the mass of the whole antibody. A preferred plasma concentration in molarity is from about 2 micromolar (μM) to about 5 millimolar (mM) and preferably, about 100 μM to 1 mM antibody antagonist. A therapeutically effective amount of an agent according of this invention which is a non-immunotherapeutic peptide or a protein polypeptide (e.g. IFN-alpha), or other similarly-sized small molecule, is typically an amount of polypeptide such that when administered in a physiologically tolerable composition is sufficient to achieve a plasma concentration of from about 0.1 microgram (μg) per milliliter (ml) to about 200 μg/ml, preferably from about 1 μg/ml to about 150 μg/ml. Based on a polypeptide having a mass of about 500 grams per mole, the preferred plasma concentration in molarity is from about 2 micromolar (μM) to about 5 millimolar (mM) and preferably about 100 μM to 1 mM polypeptide antagonist. The typical dosage of an active agent, which is a preferably a chemical antagonist or a (chemical) chemotherapeutic agent according to the invention (neither an immunotherapeutic agent nor a non-immunotherapeutic peptide/protein) is 10 mg to 1000 mg, preferably about 20 to 200 mg, and more preferably 50 to 100 mg per kilogram body weight per day. The preferred dosage of an active agent, which is a preferably a chemical antagonist or a (chemical) chemotherapeutic agent according to the invention (neither an immunotherapeutic agent nor a non-immunotherapeutic peptide/protein) is 0.5 mg to 3000 mg per patient and day, more preferably 10 to 2500 mg per patient and per day, and especially 50 to 1000 mg per patient and per day, or, per kilogram body weight, preferably about 0.1 to 100 mg/kg, and more preferably 1 mg to 50 mg/kg, preferably per dosage unit and more preferably per day, or, per square meter of the bodysurface, preferably 0.5 mg to 2000 mg/m$^2$, more preferably 5 to 1500 mg/m$^2$, and especially 50 to 1000 mg/m$^2$, preferably per dosage unit and more preferably per day.

The term "**therapeutically effective**" or "**therapeutically effective amount**" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

As used herein, the term "solvate" preferably refers to a complex of variable stoichiometry formed by a solute (in this invention, a specific integrin ligand and/or a further cancer cotherapeutic agent (or a salt or physiologically functional derivative thereof)) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water. Pharmaceutically acceptable salts of compounds to be used according to the invention and their preparation is known in the art. If the compound itself is not a salt, it can be easily transferred into a salt by addition of a pharmaceutically acceptable acid or of a pharmaceutically acceptable base. Pharmaceutically acceptable acids and bases are known in the art, for example from the literature cited herein.

The compounds for use according to the invention, the specific integrin ligand and/or at least one further cancer cotherapeutic agent different from the at least one specific integrin ligand selected from temozolomide, can generally be administered to the patient in a form and in a way or manner that is known in the art for the respective compounds or class of compounds, for example as described herein or as described in the literature cited herein.

The specific integrin ligand cyclo-(Arg-Gly-Asp-DPhe-NMeVal) is preferably applied as a pharmaceutically acceptable salt, more preferably the pharmacologically acceptable hydrochloride salt, and especially preferably applied as the inner (or internal) salt, which is the compound cyclo-(Arg-Gly-Asp-DPhe-NMeVal) as such.

With regard to the specific integrin ligand cyclo-(Arg-Gly-Asp-DPhe-NMeVal), the following kinds of writing the name are preferably to be regarded as equivalent:

cyclo-(Arg-Gly-Asp-DPhe-NMeVal) = cyclo-(Arg-Gly-Asp-DPhe-NMe-Val) =

cyclo(Arg-Gly-Asp-DPhe-NMeVal) = cyclo(Arg-Gly-Asp-DPhe-NMe-Val) =

cRGDfNMeV = c(RGDfNMeV).

Recent results show that inhibiting integrins, especially $\alpha v \beta 3$ and/or $\alpha v \beta 5$, commonly expressed in various cancerous cells, such as glioblastoma, can significantly decrease the resistance to ionising radiation of otherwise radioresistant cancerous cells and/or can induce an increased sensitivity of cancerous cells towards ionising radiation. Ionising radiation in this respect is preferably external beam radiation and especially fractionated external beam radiation.

Especially with respect to primary brain tumours, such as astrocytoma and glioblastoma multiforme, a significant percentage of the patients will die of a relapse originating in the in the radiation fields, likely as a result of low tumor sensitivity to radiation caused by an activation of the survival signalling. This increased radiation resistance (thereafter named "radioresistance") is due to the modulation of different biological signal transduction pathways and to a cross-talk between the tumor cells and their microenvironment.

Accordingly, specific integrin ligands, especially integrin ligands specific to $\alpha v \beta 3$ and/or $\alpha v \beta 5$ integrins according to the invention can be successfully applied to improve the efficacy of various cancer cotherapeutic agents and especially the efficacy of radiation therapy. Radiation therapy in this respect is preferably external beam radiation and especially fractionated external beam radiation.

Glioblastoma (GBM, Astrocytoma, WHO Grade IV), also named Glioblastomamultiforma or Glioblastoma multiforme, are invasive brain tumors with a poor prognosis, and a median survival irrespective of treatment of around 14-15 months. Generally, locally invasive tumors inevitably relapse, whether treated with surgery, chemotherapy or irradiation. The current state of the art regimen ("EORTC protocol", as established by Stupp and coworkers) uses graded RTX accompanied by the cytotoxic agent temozolomide (see especially R.Stupp et al., NEJM 2005). So far, this has extended the life span of patients by some months at best.

Glioblastoma is highly vascularized, and the angiogenic process is necessary for tumor development. Both the GBM vasculature and the GBMs themselves overexpress the integrin $\alpha_v \beta_3$. This has been shown in vitro to support the survival of GBM cell lines, as well as being a feature of angiogenic endothelia, driven into the cell cycle by tumor derived growth factors like VEGF. Inhibition of $\alpha_v \beta_3$ on endothelial cells and isolated glioblastoma cell lines induces cell death, and can activate apoptosis. RTX has also been shown to induce $\alpha_v \beta_3$ expression on endothelial cells. Together these data suggest that $\alpha_v \beta_3$ may support survival of the vascular and tumor compartment of GBM.

Cilengitide is a cyclic pentapeptide that specifically binds to alpha-v integrins, notably to $\alpha_v \beta_3$, and inhibits their activation by their ligands within the extracellular matrix. Cilengitide preferably induces apoptosis in endothelial and GBM cell cultures.

A phase I clinical study used cilengitide treatment in a dose escalation study on various brain tumors (NABT 9911). In some of the GBM patients in this study, an indication of response was seen. Cilengitide (= cyclo-(Arg-Gly-Asp-DPhe-NMe-Val), in very marked contrast to most cancer therapeutics currently in use has a very innocuous side effect profile, with no known MTD in humans - and is very well tolerated.

In addition to the essentially 100% mortality in GBM patients (2-year survival rate about 25%), the morbidity from neurological complications also rapidly degrades the quality of life (QOL).

Thus, it is an objective of the instant invention to provide a more effective, and better tolerated treatment for GBM patients leading to enhanced QOL and increased median survival, preferably as well in GBM patients following 1st relapse as in the first line treatment of GBM patients.

Thus, the application describes a method of treatment of GBM, comprising Cilengitide administration by i.v. infusion in GBM patients, preferably GBM patients following 1st relapse, more preferably newly diognosed GBM patients (1st line treatment), preferably until tumor progression, stable disease or healing occurs. In this method of treatment, the Cilengitide is preferably administered twice weekly (e.g. q3q4), preferably in an ca. 1 h i.v. infusion, preferably in infusion saline, and preferably either at 500 mg flat dose or 2000 mg flat dose.

Advantagously, MRI and neurological indications of objective tumor response to this method of treatment can be shown in at least 20% of the pathologically confirmed GBM patients under therapy.

This method of treatment can optionally be combined, partially ot totally, with administration of one or more cancer cotherapeutic agents, preferably as described herein.

Moreover, due to the synergistic, efficacy enhancing properties of the specific integrin ligand Cilengitide with various cancer cotherapeutic agents, the combination of Cilengitide, at least one chemotherapeutic and external beam radiation is described.

For example, the standard of treatment of glioblastoma multiforme, associating radiotherapy and temozolomide, has only increased the median survival of resected patients by 2.5 months (12.1 → 14.6 months) compared to radiotherapy alone (Stupp et al., 2005). However, in combination with at least one specific integrin ligand for use according to the invention selected from cyclo-(Arg-Gly-Asp-DPhe-NMe-Val) , this standard treatment shows significantly improved efficacy with respect to an increased median survival and quality of life. Accordingly, this combination of radiotherapy, temozolomide and at least one specific integrin ligand as described herein, is the aspect of the instant invention.

Thus, combinations of at least one specific integrin ligand as described herein and at least one cancer cotherapeutic agent as described herein, can be effectively used to treat intracerebral tumor growth in the brain of a host. Intracerebral tumor growth in the brain of a host includes, but is not limited to primary brain tumours, astrocytoma, such as astrocytoma grade I-IV (WHO), and especially glioblastoma multiforme. Intracerebral tumor growth is preferably also including intercerebral metastases of other cancer types, preferably cancer types selected from small cell lung cancer and non-small cell lung cancer, preferably non-small cell lung cancer, breast cancer, metastatic melanoma, metastatic androgen independent prostate cancer, metastatic androgen dependent prostate cancer, and more preferably from breast cancer, small cell lung cancer and especially non-small cell lung cancer.

Preferred subject of the instant invention is the use of at least one specific integrin ligand, selected from cyclo-(Arg-Gly-Asp-DPhe-NMeVal) and the pharmaceutically acceptable solvates and salts thereof, for the manufacture of a medicament for the treatment of astrocytoma, wherein the medicament is to be used in combination with temozolomide and radiotherapy, and wherein the astrocytoma is glioblastoma or glioblastoma multiforme.

Preferred is a use as described herein, wherein the radiotherapy is fractionated external beam radiation.

Preferred is a use as described herein, wherein said specific integrin ligand is applied substantially concurrently or sequentially to the temozolomide and/or the radiotherapy.

Preferred is a use as described herein, wherein the at least one integrin ligand is cyclo-(Arg-Gly-Asp-DPhe-NMeVal).

Preferred is a use as described herein, wherein at least one of the specific integrin ligands is to be administered 1 to 8 hours (h) prior to the application of the radiotherapy and/or the temozolomide.

More preferred is a use as described herein, wherein at least one of the specific integrin ligands is to be administered 2 to 6 hours prior to the application of the radiotherapy and/or the temozolomide.

Especially preferred is the use of at least one specific integrin ligand, comprising cyclo-(Arg-Gly-Asp-DPhe-NMe-Val), the pharmaceutically acceptable solvates and/or salts thereof, for the manufacture of a medicament for the treatment of astrocytoma, wherein the medicament is to be used in combination with radiotherapy, wherein at least the specific integrin ligand cyclo-(Arg-Gly-Asp-DPhe-NMe-Val), the pharmaceutically acceptable solvates and/or salts thereof is to be administered 1 to 10 hours prior to the application of the radiotherapy, and wherein at least one cancer cotherapeutic agent comprising temozolomide is to be applied.

Especially preferred is the use of at least one specific integrin ligand, comprising cyclo-(Arg-Gly-Asp-DPhe-NMe-Val), the pharmaceutically acceptable solvates and/or salts thereof, for the manufacture of a medicament for the treatment of glioblastoma multiforme, wherein the medicament is to be used in combination with temozolomide and radiotherapy.

Especially preferred is the use wherein the radiotherapy is external beam radiation.

Preferred is a use as described herein, wherein at least the specific integrin ligand is to be applied 1 to 32 hours before the application of one or more further cancer cotherapeutic agents and/or radiotherapy.

Especially preferred is the use of at least one specific integrin ligand, comprising cyclo-(Arg-Gly-Asp-DPhe-NMe-Val), the pharmaceutically acceptable solvates and/or salts thereof, for the manufacture of a medicament for the treatment of astrocytoma, wherein the medicament is to be used in combination with temozolomide and radiotherapy, and wherein at least the specific integrin ligand cyclo-(Arg-Gly-Asp-DPhe-NMe-Val), the pharmaceutically acceptable solvates and/or salts thereof is to be administered to a patient in an amount of 800 mg to 7000 mg per week.

Preferred is a use as described herein, wherein cyclo-(Arg-Gly-Asp-DPhe-NMeVal) is to be administered to a patient in an amount of about 1000 mg per week, about 1500 mg per week, about 2500 mg per week, about 4000 mg per week or about 6000 mg per week.

More preferred is a use as described herein, wherein the amount of about 1000 mg per week or about 4000 mg per week is to be administered in a twice weekly administration scheme, and the amount of about 1500 mg per week or about 6000 mg per week is to be administered in a three times weekly administration scheme.

Alternatively preferred is a use as described herein, wherein the amount of about 1000 mg per week is to be administered in a twice weekly administration scheme consisting of about 500 mg per administration or the amount of about 4000 mg per week is to be administered in a twice weekly administration scheme of about 2000 mg per administration.

Especially preferred is a use as described herein, wherein at least the specific integrin ligand cyclo-(Arg-Gly-Asp-DPhe-NMe-Val), the pharmaceutically acceptable solvates and/or salts thereof is to be administered 2 to 8 hours or 3 to 8 hours prior to the application of the radiotherapy.

Further preferred is the use of at least one specific integrin ligand, comprising cyclo-(Arg-Gly-Asp-DPhe-NMe-Val), the pharmaceutically acceptable solvates and/or salts thereof, for the manufacture of a medicament for the treatment of astrocytoma, wherein the medicament is to be used in combination with radiotherapy, wherein at least the specific integrin ligand cyclo-(Arg-Gly-Asp-DPhe-NMe-Val), the pharmaceutically acceptable solvates and/or salts thereof is to be administered 1 to 10 hours prior to the application of the radiotherapy, wherein at least one cancer cotherapeutic agent comprising temozolomide is to be applied, and wherein additionally a further cancer cotherapeutic agent is to be applied, selected from Cisplatin, Oxaliplatin, Carboplatin, 5-FU, Darcabarzine, Procarbazine, Vinblastin, Vincristine, Irinotecan, Taxol, Paclitaxel, Docetaxel, Gemcitabine, Gleevec, Iressa, Tarceva and Nexavar, Herceptin, Bevacizumab, Cetuximab, Nimotuzumab, Sorafenib, Sunitinib and ZD6474 (ZACTIMA™), and even more preferably selected from Cisplatin, Oxaliplatin, Vinblastin, Taxol, Gemcitabine, Gleevec and Iressa. In this embodiment, the radiotherapy is preferably external beam radiation. In this embodiment, at least the specific integrin ligand is preferably to be applied 1 to 32 hours before the application of one or more further cancer cotherapeutic agents and/or radiotherapy. In this embodiment, at least the specific integrin ligand cyclo-(Arg-Gly-Asp-DPhe-NMe-Val), the pharmaceutically acceptable solvates and/or salts thereof is preferably to be administered to a patient in an amount of 800 mg to 7000 mg per week. In this embodiment, cyclo-(Arg-Gly-Asp-DPhe-NMeVal) is preferably to be administered to a patient in an amount of about 1000 mg per week, about 1500 mg per week, about 2500 mg per week, about 4000 mg per week or about 6000 mg per week. In this embodiment, the amount of about 1000 mg per week or about 4000 mg per week is preferably to be administered in a twice weekly administration scheme, and the amount of about 1500 mg per week or about 6000 mg per week is preferably to be administered in a three times weekly administration scheme. In this embodiment, the amount of about 1000 mg per week is preferably to be administered in a twice weekly administration scheme consisting of about 500 mg per administration or the amount of about 4000 mg per week is preferably to be administered in a twice weekly administration scheme of about 2000 mg per administration. In this embodiment, at least the specific integrin ligand cyclo-(Arg-Gly-Asp-DPhe-NMe-Val), the pharmaceutically acceptable solvates and/or salts thereof is preferably to be administered 2 to 8 hours or 3 to 8 hours prior to the application of the radiotherapy.

Thus, the invention also refers to the use of

(a composition containing) at least one specific integrin ligand, selected from and cyclo-(Arg-Gly-Asp-DPhe-NMe-Val),

for the manufacture of a medicament for the treatment of astrocytoma, wherein the medicament is to be used in combination with the two further cancer cotherapeutic agents, temozolomide and radiotherapy, more preferably external beam radiation and especially fractionated external beam radiation. The specific integrin ligand is preferably to be applied substantially concurrently or sequentially to at least one of the cancer cotherapeutic agents.

Accordingly, one preferred aspect of the instant invention relates to the use of (a composition containing) at least one specific integrin ligand, comprising cyclo-(Arg-Gly-Asp-DPhe-NMe-Val),

for the manufacture of a medicament for the treatment of glioblastoma multiforme, wherein the medicament is to be used in combination with the two further cancer cotherapeutic agents, temozolomide and radiotherapy, preferably temozolomide and external beam radiation and especially temozolomide and fractionated external beam radiation. Also in this preferred aspect, at least the specific integrin ligand is preferably to be applied timed as described herein.

Especially preferably, the invention also refers to the use of

(a composition containing) at least one specific integrin ligand, selected from and cyclo-(Arg-Gly-Asp-DPhe-NMe-Val),

for the manufacture of a medicament for the treatment of astrocytoma, wherein the medicament is to be used in combination with the two further cancer cotherapeutic agents, temozolomide and radiotherapy, preferably more preferably external beam radiation and especially fractionated external beam radiation, wherein at least one of the specific integrin ligands is to be administered in a timed administration as described herein, preferably 1 to 8 hours (h), preferably 2 to 6 h, and most preferably 2 to 4 h prior to the application of the radiotherapy and/or the temozolomide.

Even more preferably, the invention also refers to the use of

a) (a composition containing) at least one specific integrin ligand, and

b) further cancer cotherapeutic agents different from the at least one specific integrin ligand of a)

for the manufacture of a medicament for the treatment glioblastoma, preferably glioblastoma multiforme, in human animals,

wherein the at least one specific integrin ligand is cyclo-(Arg-Gly-Asp-DPhe-NMe-Val), and the further cancer cotherapeutic agents different from the at least one specific integrin ligand of a) are radiotherapy, preferably external beam radiation, and temozolomide,

wherein a) is to be administered 2 to 32 hours (h), preferably 4 to 24 h, more preferably 6 to 20 h and most preferably 6 to 16 h prior to the application of b).

Especially preferably, the invention also refers to the use of

(a composition containing) at least one specific integrin ligand, selected from cyclo-(Arg-Gly-Asp-DPhe-NMe-Val), the pharmaceutically acceptable solvates and salts thereof, and especially cyclo-(Arg-Gly-Asp-DPhe-NMe-Val),

for the manufacture of a medicament for the treatment of astrocytoma, wherein the medicament is to be used in combination with the two further cancer cotherapeutic agents, temozolomide and radiotherapy, preferably temozolomide and external beam radiation and especially temozolomide and fractionated external beam radiation, wherein at least one of the specific integrin ligands is to be administered in a timed administration as described herein, preferably 1 to 8 hours (h), preferably 2 to 6 h, and most preferably 2 to 4 h prior to the application of the radiotherapy and/or the temozolomide.

Especially preferably, the invention also refers to the use of

(a composition containing) at least one specific integrin ligand, selected from cyclo-(Arg-Gly-Asp-DPhe-NMe-Val) and the pharmaceutically acceptable salts thereof, for the manufacture of a medicament for the treatment of astrocytoma, wherein the medicament is to be used in combination with the two further cancer cotherapeutic agents, temozolomide and radiotherapy, preferably temozolomide and external beam radiation and especially temozolomide and fractionated external beam radiation, wherein at least one of the specific integrin ligands is to be administered 2 to 32 hours (h), preferably 4 to 24 h, more preferably 6 to 20 h and most preferably 6 to 16 h prior to the application of the radiotherapy and/or the temozolomide.

Especially preferably, the invention also refers to the use of

(a composition containing) at least one specific integrin ligand, comprising cyclo-(Arg-Gly-Asp-DPhe-NMe-Val),

for the manufacture of a medicament for the treatment of glioblastoma multiforme, wherein the medicament is to be used in combination with the two further cancer cotherapeutic agents, temozolomide and radiotherapy, preferably temozolomide and external beam radiation and especially temozolomide and fractionated external beam radiation, wherein at least the specific integrin ligand cyclo-(Arg-Gly-Asp-DPhe-NMe-Val) is to be administered in a timed administration as described herein, preferably 1 to 24 hours (h), preferably 1 to 20 h, more preferably, 2 to 20 h, more preferably 2 to 16 h, 3 to 16, even more preferably 3 to 12 h and especially 4 to 10 h prior to the application of the radiotherapy and/or the temozolomide.

Especially preferably, the invention also refers to the use of

(a composition containing) at least one specific integrin ligand, comprising cyclo-(Arg-Gly-Asp-DPhe-NMe-Val),

for the manufacture of a medicament for the treatment of glioblastoma multiforme, wherein the medicament is to be used in combination with the two further cancer cotherapeutic agents, temozolomide and radiotherapy, preferably temozolomide and external beam radiation and especially temozolomide and fractionated external beam radiation, wherein at least the specific integrin ligand cyclo-(Arg-Gly-Asp-DPhe-NMe-Val) is to be administered in a timed administration as described herein, preferably 1 to 10 hours (h), preferably 2 to 8 h, more preferably 2 to 6 h, even more preferably 3 to 8 h, even more preferably 3 to 6 h and especially 4 to 8 h prior to the application of the radiotherapy and/or the temozolomide.

Recent *in vitro* results show an increase in cell death/deterioriation after combination treatment of lung cancer cell lines, such as A549, H157, H322, H460 and/or H1975, with specific integrin ligands, such as Vitaxin, Abegrin, CNTO95 and cyclo-(Arg-Gly-Asp-DPhe-NMe-Val), and cancer cotherapeutic agents, such as Cisplatin, Oxaliplatin, Vinblastin, Taxol, Gemcitabine, Gleevec, Iressa, and radiotherapy, preferably external beam radiation and/or fractionated external beam radiation. The results suggest that cancer cotherapeutic agents, such as radiation, can induce expression of relevant integrins in lung cancer cells, and/or that the specific integrin ligand is acting as an amplifier of efficacy, e.g. as a radio amplifier. Moreover, combined application of at least one specific integrin ligand and at least one cancer cotherapeutic agent, preferably radiation, results in significant cell kill and thus reduced survival curves of the respective treated cells considerably. Accordingly, the combinations appear to effectively induce cell death, likely due to apoptosis and/or mitotic cell death, in endothelial cells and tumour cells, especially in lung cancer cells and especially in non-small cell lung cancer cells. The extent of effect may depend on the degree of target expression, i.e. integrin expression. Thus, combinations of at least one specific integrin ligand as described herein and at least one cancer cotherapeutic agent as described herein, can be effectively used to treat lung cancer, and especially small cell lung cancer, non-small cell lung cancer and/or metastases thereof.

Subject of the instant invention is the use of at least one specific integrin ligand, comprising cyclo-(Arg-Gly-Asp-DPhe-NMeVal) and/or the pharmaceutically acceptable salts thereof, for the manufacture of a medicament for the treatment of astrocytoma, wherein the medicament is to be used in combination with radiotherapy, preferably external beam radiation, wherein at least the specific integrin ligand cyclo-(Arg-Gly-Asp-DPhe-NMeVal) and/or the pharmaceutically acceptable salts thereof is to be administered to a patient in an amount of about 1000 mg per week or about 4000 mg per week, combined with one or more further cancer cotherapeutic agents, comprising Temozolomide.

Subject of the instant invention is the use of at least one specific integrin ligand, comprising cyclo-(Arg-Gly-Asp-DPhe-NMeVal) and/or the pharmaceutically acceptable salts thereof, for the manufacture of a medicament for the treatment of astrocytoma, wherein the medicament is to be used in combination with temozolomide and radiotherapy, preferably external beam radiation, wherein at least the specific integrin ligand cyclo-(Arg-Gly-Asp-DPhe-NMeVal) and/or the pharmaceutically acceptable salts thereof is to be administered to a patient in an amount of 800 mg to 7000 mg per week.

Preferably, the amount of cyclo-(Arg-Gly-Asp-DPhe-NMeVal) and/or a pharmaceutically acceptable salt thereof, preferably cyclo-(Arg-Gly-Asp-DPhe-NMeVal), to be administered to a patient per week is to be administered in about equal amounts of about 500 mg or about 2000 mg for each administration.

More preferably, the amount of cyclo-(Arg-Gly-Asp-DPhe-NMeVal) and/or a pharmaceutically acceptable salt thereof, preferably cyclo-(Arg-Gly-Asp-DPhe-NMeVal), is to be administered to a patient in an amount of about 1000 mg per week, about 1500 mg per week, about 2500 mg per week, about 4000 mg per week or about 6000 mg per week.

Preferably, the amount of about 1000 mg of cyclo-(Arg-Gly-Asp-DPhe-NMeVal) and/or a pharmaceutically acceptable salt thereof, preferably of is cyclo-(Arg-Gly-Asp-DPhe-NMeVal), per week is to be administered in a twice weekly administration scheme.

Preferably, the amount of about 4000 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is to be administered in a twice weekly administration scheme, preferably in about equal amounts of about 2000 mg each.

In the twice weekly administration scheme, the administration is preferably done on a day one and then on day three or a day four. Thus, the twice weekly administration scheme is preferably done either in an alternating every third day/every fourth day scheme or an alternating every fourth day/every third day scheme, such as an administration on mondays and thursdays (as an example of the 3/4 scheme) or tuesdays and fridays (as a further example of the 3/4 scheme), or on Thursdays and Mondays (as an example of the 4/3 scheme) or on Fridays and Tuesdays (as a further example of the 4/3 scheme).

The twice weekly administration scheme, preferably the twice weekly administration scheme as described above, can be applied to the patient once or several times. Preferably, it is applied several times, even more preferably at least three times or at least six times. For example, the twice weekly administration scheme can be applied continuously until healing, stable disease or tumor progression takes place. Typically, the twice weekly administration scheme, preferably the twice weekly administration scheme as described above, is applied 4 to 156 times, such as about 4 times, about 8 times, about 16 times, about 24 times, about 35 times, about 70 times or about 104 times.

The twice weekly administration scheme can be combined partially or totally with radiotherapy, preferably radiotherapy as described herein. Preferably, the twice weekly administration scheme is combined partially with radiotherapy.

Preferably, the amount of about 1500 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is to be administered in a three times weekly administration scheme, preferably in about equal amounts of about 500 mg each.

Preferably, the amount of about 6000 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is to be administered in a three times weekly administration scheme, preferably in about equal amounts of about 2000 mg each.

In the three times weekly administration scheme, the administration is preferably either done on a day one, on a day three or a day four and then on a day 6, or more preferably on a day one, on a day 3 and on a day 5, then followed of two consecutive days off. The latter three times weekly administration scheme, for example, typically starts on a monday, followed by one administration on the following wednesday and one administration on friday, with saturday and sunday off of treatment.

The three times weekly administration scheme, preferably the three times weekly administration scheme as described above, can be applied to the patient once or several times. Preferably, it is applied several times, even more preferably at least three times or at least six times. For example, the three times weekly administration scheme can be applied continuously till healing or tumor progression takes place. Typically, the twice weekly administration scheme, preferably the twice weekly administration scheme as described above, is applied 4 to 156 times, such as about 4 times, about 8 times, about 16 times, about 24 times, about 35 times, about 70 times or about 104 times.

The three times weekly administration scheme can be combined partially or totally with radiotherapy, preferably radiotherapy as described herein. Preferably, the three times weekly administration scheme is combined partially with radiotherapy.

Preferably, the amount of about 2500 mg of cyclo-(Arg-Gly-Asp-DPhe-NMeVal) and/or a pharmaceutically acceptable salt thereof, preferably of cyclo-(Arg-Gly-Asp-DPhe-NMeVal), per week is to be administered in a five times weekly administration scheme, preferably in about equal amounts of about 500 mg each. In the five times weekly administration scheme, the administration is preferably done on five consecutive days, preferably followed by 2 days in a row off. This "5 days of consecutive administration followed by 2 consecutive days off" scheme can be repeated once or several times. Preferably, this before described "5 days of consecutive administration followed by 2 consecutive days off' scheme is performed more than once but preferably less than 18 times, more preferably 2 to 12 times, even more preferably 3 to 8 times and especially 4 to 6 times, for example 2 times, 3 times, 4 times, 5 times, 6 times, 8 times or 12 times. Especially preferably, this "5 days of consecutive administration followed by 2 consecutive days off' scheme is applied 6 times.

Preferably, this "5 days of consecutive administration followed by 2 consecutive days off" scheme is combined with radiotherapy as described herein, preferably radiotherapy as described herein that is applied to the patient in an analog "5 days of consecutive application followed by 2 consecutive days off" scheme that preferably runs in parallel to the other scheme, preferably with the same two days off.

Regarding the herein described weekly administation amounts and/or schemes, the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is preferably administered in a timed administration as described herein, generally 1,5 to 20 hours (h), preferably 2 to 16 h, more preferably 2 to 12 h, even more preferably 2 to 10 h, even more preferably 3 to 10 h and especially 2 to 8 h prior to the application of the radiotherapy. Alternatively, the specific integrin ligand cyclo-(Arg-Gly-Asp-DPhe-NMeVal) and/or the pharmaceutically acceptable salts thereof is administered in a timed administration as described herein, preferably 1 to 10 hours (h), preferably 1 to 6, more preferably 2 to 8, even more preferably 3 to 8 h, even more preferably 3 to 6 and especially 4 to 8 h prior to the application of the radiotherapy.

According to the invention, the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), and optionally the at least one further cancer cotherapeutic agent different from the at least one specific integrin ligand selected from Temozolomide, , and radiotherapy, preferably radiotherapy as described herein, is preferably to be administered to a patient in need thereof in a dose/mode of administration/dosing schedule given below:

The specific integrin ligand cyclo-(Arg-Gly-Asp-DPhe-NMeVal) and/or the pharmaceutically acceptable salts thereof, preferably cyclo-(Arg-Gly-Asp-DPhe-NMeVal) is preferably administered at a flat dose of about 500 mg or about 2000 mg per administration, preferably in a weekly administration scheme selected from a twice weekly administration scheme, three times weekly administration scheme and five times weekly administration scheme, preferably as described herein, more preferably in the twice weekly administration scheme as described herein, which adminstration scheme is preferably repeated at least once, more preferably at least twice, more preferably at least 5 times and even more preferably at least 35 times. Preferably, it is repeated less than 150 times, more preferably less than 100 times, at least if it is administered without a pause, break or gap of at least one week, preferably of at least four weeks. After such a pause, gap or break, the above described administration scheme can be repeated once or several times, if necessary. Accordingly, the afore described administration scheme is preferably applied to the patient for at least 2 weeks, preferably at least 6 weeks, more preferably at least 12 weeks, even more preferably at least 24 weeks, and especially at least 35 weeks, for example for about 4 weeks, for about 6 weeks, for about 35 weeks, for about 36 weeks, for about 72 weeks or for about 120 weeks. In the afore described administration scheme, the specific integrin ligand cyclo-(Arg-Gly-Asp-DPhe-NMVal) and/or the pharmaceutically acceptable salts thereof, preferably cyclo-(Arg-Gly-Asp-DPhe-NMeVal), is preferably administered. In the afore described administration scheme, the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is preferably administered by i. v. infusion twice a week, preferably on Day 1 and 4, e. g., Monday and Thursday or Tuesday and Friday), preferably for 35 weeks or at least 35 weeks without a pause. In the afore described administration scheme, the adminstration can be continued until progression, stable disease or healing occurs. Preferably, the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), begins on Day 1 of each week.

Preferably, the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is combined, partially or totally, preferably partially, with the administration of at least one further cancer cotherapeutic agent different from the at least one specific integrin ligand, wherein the further cancer cotherapeutic agent is selected from Temozolomide,

Preferably, the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is combined, partially or totally, preferably partially, with the administration or delivery of radiotherapy, preferably external beam radiation and especially focal radiotherapy. Preferably, the radiotherapy is administerd or delivered on one or more days within one or more weeks in which the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is administered in a weekly administation scheme, preferably a weekly administation scheme as described herein. More preferably, the radiotherapy is administerd or delivered on one or more days within one or more weeks in which the specific integrin ligand cyclo-(Arg-Gly-Asp-DPhe-NMeVal) and/or the pharmaceutical acceptable salts thereof, preferably cyclo-(Arg-Gly-Asp-DPhe-NMeVal) is administered in a twice weekly administation scheme. The radiotherapy, preferably external beam radiation and especially focal radiotherapy, is preferably administerd or delivered over a period from 1 to 12 weeks, preferably 2 to 10 weeks, more preferably 3 to 8 weeks and especially 4 to 6 weeks, for example about 3 weeks, about 5 weeks, about 6 weeks, about 7 weeks or about 9 weeks, with an administration or delivery on 1 to 7 days, preferably 2 to 6 days and especially 3 to 5 days, for example, 2 days, 3 days, 5 days or 7 days, per week.

Preferably, the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is combined, partially or totally, preferably partially, with the administration or delivery of focal radiotherapy, wherein 40 to 70 Gray (Gy), preferably 50 to 66 Gy, more preferably 55 to 62 Gy, for example about 58 Gy, about 60 Gy or about 65 Gy are administered or delivered to the patient, preferably in fractions of 0.5 to 5 Gy, more preferably 1 to 3 Gy and especially 1.5 to 2.5 Gy, for example about 1.3 Gy, about 1.6 Gy, about 1.8 Gy, about 2.0 Gy or about 2.2 Gy, per per administration or delivery, which is preferably also the amount of radiation per day on which the administration or delivery of the radiation takes place. Accordingly, an administration or delivery of 1.5 to 2.5 Gy and preferably 1.8 to 2.2 Gy per day for 5 days within one week, even more preferably 5 consecutive days within one week, is preferred. The kind of application of focal radiotherapy as described above is preferred in the treatment of primary brain tumors, including astrocytoma, preferably astrocytoma grade III and/or grade IV, and especially GBM.

Alternatively, the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), is combined, partially or totally, preferably partially, with the administration or delivery of focal radiotherapy, wherein 20 to 50 Gray (Gy), preferably 25 to 40 Gy, more preferably 28 to 25 Gy, for example about 28 Gy, about 30 Gy or about 35 Gy are administered or delivered to the patient, preferably in fractions of 0.5 to 5 Gy, more preferably 0.8 to 3 Gy and especially 1 to 2.5 Gy, for example about 1.0, about 1.3 Gy, about 1.6 Gy, about 1.8 Gy, about 2.0 Gy, about 2.5 Gy or about 3.0 Gy, per per administration or delivery, which is preferably also the amount of radiation per day on which the administration or delivery of the radiation takes place. Accordingly, an administration or delivery of 1.5 to 2.5 Gy and preferably 1.8 to 2.2 Gy per day for 2 or 3 days within one week is preferred. Accordingly, an administration or delivery of 0.7 to 1.3 Gy and preferably 0.9 to 1.2 Gy per day for 3 to 6 days, preferably for 5 days and more preferably 5 consecutive days, within one week, is also preferred. Generally, the administration or delivery of 1.0 to 3.0 Gy, preferably about 1.0, about 2.0 Gy or about 3.0 Gy per day for 2 or 3 days within one week is especially preferred. The kind of application of focal radiotherapy as described above is preferred in the treatment of brain metastases, preferably brain metastases of cancer types selected from the group consisting of small cell lung cancer and non-small cell lung cancer, preferably non-small cell lung cancer, breast cancer, metastatic melanoma, metastatic androgen independent prostate cancer, metastatic androgen dependent prostate cancer.

Typically, both the amounts of about 30 Gy and about 60 Gy are administered or delivered to the patient within about six consecutive weeks.

An especially preferred subject of the instant invention relates to Cyclo-(Arg-Gly-Asp-DPhe-NMe-Val) for use in a method for the treatment of astrocytoma in combination with temozolomide and radiotherapy.

Preferably, the astrocytoma is glioblastoma or glioblastoma multiforme.

Preferably, the radiotherapy is fractionated external beam radiation.

Especially preferably, said Cyclo-(Arg-Gly-Asp-DPhe-NMe-Val) is applied substantially concurrently or sequentially to the temozolomide and/or the radiotherapy.

Even more preferably, said Cyclo-(Arg-Gly-Asp-DPhe-NMe-Val) is administered 1 to 8 hours (h) prior to the application of the radiotherapy and/or the temozolomide.

Even more preferably, said Cyclo-(Arg-Gly-Asp-DPhe-NMe-Val) is administered 2 to 6 hours prior to the application of the radiotherapy and/or the temozolomide.

Accordingly, preferred is a method of treatment (A), comprising per patient within one week

a) the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), in a twice weekly administration schedule comprising about 500 mg or about 2000 mg (flat) of cyclo-(Arg-Gly-Asp-DPhe-NMeVal) per administration; and

b) the once daily administration or delivery of fractionated focal radiotherapy at 1.5 to 2.5 Gy per fraction, on 2 to 7 days, preferably 3 to 6 days, more preferably 5 days and especially preferably on 5 consecutive days within one week;

wherein said treatment is preferably applied to the patient for at least 2 weeks, more preferably for at least 2 consecutive weeks, even more preferably for at least 4 consecutive weeks and especially for 6 or more consecutive weeks, but generally for less than 13 consecutive weeks, preferably less then 11 consecutive weeks and even more preferably less than 9 consecutive weeks, for example an application for 2 consecutive weeks, 4 consecutive weeks, 5 consecutive weeks, 6 consecutive weeks, 7 consecutive weeks or 10 consecutive weeks.

With respect to this method, said specific integrin ligand is preferably administered on days on which the fractionated focal radiotherapy is administered or delivered to the patient, more preferably administered in a timed administration as described herein. With respect to this method, said specific integrin ligand is preferably administered in a timed administration as described herein, more preferably 1,5 to 20 hours (h), preferably 2 to 16 h, more preferably 2 to 12 h, even more preferably 2 to 10 h, even more preferably 3 to 10 h and especially 2 to 8 h prior to the application of the radiotherapy.

The afore described method of treatment is especially advantageous in the treatment of tumors or cancer selected from astrocytoma, preferably astrocytoma grade II, III and/or IV, glioblastoma, preferably glioblastoma multiforme.

The afore described method of treatment can optionally be combined with the additional administration of one or more specific integrin ligands as described herein other than cyclo-(Arg-Gly-Asp-DPhe-NMeVal) and/or the pharmaceutically acceptable salts thereof, preferably specific integrin ligands selected from the group consisting of LM609, Vitaxin, Abegrin, Abciximab, P1F6, 14D9.F8 and CNT095, more preferably Vitaxin, Abegrin, CNT095 and Abciximab), and/or the additional administration of at least one cancer cotherapeutic agent as described herein other than radiotherapy, preferably at least one cancer cotherapeutic agent selected from the group consisting of Cisplatin, Oxaliplatin, Carboplatin, 5-FU, Darcabarzine, Procarbazine, Vinblastin, Vincristine, Irinotecan, Paclitaxel, Docetaxel, Gemcitabine, Gleevec, Iressa, Tarceva and Nexavar, even more prefrably preferably selected from Cisplatin, Oxaliplatin, Vinblastin, Taxol, Gemcitabine, Gleevec and Iressa, and/or selected from the group consisting Herceptin, Bevacizumab (rhuMAb-VEGF, Avastin®), Cetuximab (Erbitux®) and Nimotuzumab, and preferably Sorafenib (Nexavar®), Sunitinib (Sutent®) and ZD6474 (ZACTIMA™),

Accordingly, preferred is a method of treatment (B), comprising per patient within one week

a) the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), in a twice weekly administration schedule comprising about 500 mg or about 2000 mg (flat) of cyclo-(Arg-Gly-Asp-DPhe-NMeVal) per administration; and

b) the once daily administration or delivery of fractionated focal radiotherapy at 1.5 to 2.5 Gy per fraction, preferably on 2 to 5 days within one week, more preferably on 5 consecutive days within one week;

c) the administration of temozolomide on 2 to 7 days, preferably 3 to 6 days, more preferably 5 days and especially preferably on 5 consecutive days within one week, preferably in an amount per day of 25 mg/m$^2$ to 250 mg/m$^2$, more preferably 50 mg/m$^2$ to 150 mg/m$^2$, even more preferably 65 mg/m$^2$ to 100 mg/m$^2$, and especially about 75 mg/m$^2$;

wherein said method of treatment is preferably applied to the patient for at least 2 weeks, more preferably for at least 2 consecutive weeks, even more preferably for at least 4 consecutive weeks and especially for 6 or more consecutive weeks, but generally for less than 13 consecutive weeks, preferably less then 11 consecutive weeks and even more preferably less than 9 consecutive weeks, for example it is applied for 2 consecutive weeks, 4 consecutive weeks, 5 consecutive weeks, 6 consecutive weeks, 7 consecutive weeks or 10 consecutive weeks.

With respect to this method, said specific integrin ligand is preferably administered on days on which the fractionated focal radiotherapy is administered or delivered to the patient, more preferably administered in a timed administration as described herein. With respect to this method, said specific integrin ligand is more preferably administered in a timed administration as described herein, more preferably 1,5 to 20 hours (h), preferably 2 to 16 h, more preferably 2 to 12 h, even more preferably 2 to 10 h, even more preferably 3 to 10 h and especially 2 to 8 h prior to the application of the radiotherapy. With respect to this method, the temozolomide is preferably administered orally, preferably 15 to 300 minutes, more preferably 30 to 180 minutes, even more preferably 45 to 90 minutes and especially about one hour or within one hour before the administration or delivery of the radiotherapy. This administration scheme for cancer cotherapeutic agents other than radiotherapy, selected from temozolomide, is preferably also suitable for the other methodes or uses as described herein.

The afore described method of treatment is especially advantageous in the treatment of tumors or cancer as described herein, preferably tumors or cancer selected from the group consisting of primary brain tumours, astrocytoma, preferably astrocytoma grade II, III and/or IV, glioblastoma, preferably glioblastoma multiforme, and brain metastases of other cancer types, preferably selected from the group consisting of small cell lung cancer and non-small cell lung cancer, preferably non-small cell lung cancer, breast cancer, metastatic melanoma, metastatic androgen independent prostate cancer, metastatic androgen dependent prostate cancer.

Accordingly, preferred is a method of treatment (C), comprising per patient

a) optionally the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), preferably as a single agent, in a twice weekly administration schedule comprising about 500 mg or about 2000 mg (flat) of cyclo-(Arg-Gly-Asp-DPhe-NMeVal) per administration, wherein the weekly administration schedule is applied to patient for at least one week, preferably 1 to 12 weeks, more preferably 1 to 6 weeks, even more preferably 1 to 3 weeks and especially 1 or 2 weeks; followed by, preferably in the consecutive week(s),

b) the administration of temozolomide on 2 to 7 days, preferably 3 to 6 days, more preferably 5 days and especially preferably on 5 consecutive days, within one week, preferably in an amount per day of 50 mg/m$^2$ to 350 mg/m$^2$, more preferably 75 mg/m$^2$ to 250 mg/m$^2$, even more preferably 150 mg/m$^2$ to 250 mg/m$^2$, and especially about 200 mg/m$^2$; preferably combined within said week with the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), in a twice weekly administration schedule comprising about 500 mg or about 2000 mg (flat) of cyclo-(Arg-Gly-Asp-DPhe-NMeVal) per administration,

wherein step b) is applied to the patient at least once, preferably 1 to 12 weeks consecutively, more preferably 1 to 6 weeks consecutively, even more preferably 1 to 3 weeks consecutively and especially one week or 2 weeks consecutively;

and wherein said specific integrin ligand cyclo-(Arg-Gly-Asp-DPhe-NMeVal) and/or the pharmaceutically acceptable salts thereof, preferably cyclo-(Arg-Gly-Asp-DPhe-NMeVal) is preferably administered on days where the temozolomide is also administered; optionally followed by, preferably in the consecutive week(s),

c) the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), preferably as a single agent, in a twice weekly administration schedule comprising about 500 mg or about 2000 mg (flat) of cyclo-(Arg-Gly-Asp-DPhe-NMeVal) per administration, wherein the weekly administration schedule is applied to patient for at least one week, preferably 1 to 12 weeks, more preferably 2 to 6 weeks, even more preferably 2 to 4 weeks and especially 3 weeks.

Preferably, said method of treatment is preferably applied to the patient at least once, more preferably at least two times, even more preferably at least four times and especially preferably six or more times, preferably six times, but generally less than 13 times, preferably less than 9 times, even more preferably less than seven times, for example 3 times, 5 times, 6 times, 7 times or 6 times, preferably in a consecutive row, i.e. without one or more pauses in between the repetition of the method.

With respect to this method, said specific integrin ligand is more preferably administered in a timed administration as described herein, more preferably 1,5 to 20 hours (h), preferably 2 to 16 h, more preferably 2 to 12 h, even more preferably 2 to 10 h, even more preferably 3 to 10 h and especially 2 to 8 h prior to the application of the radiotherapy. With respect to this method, the temozolomide is preferably administered orally, preferably in analog manner as described herein for the timed administration of the specific integrin ligand, or preferably within 5 h, preferably within 3 h and especially within 1 h before or after, preferably after, the delivery of the radiotherapy.

The afore described method of treatment (C) is especially advantageous in the treatment of tumors or cancer selected from astrocytoma, preferably astrocytoma grade II, III and/or IV, glioblastoma, preferably glioblastoma multiforme.

Accordingly, preferred is a method of treatment (D), comprising per patient

a) within one first week: the administration of temozolomide on 2 to 7 days, preferably 3 to 6 days, more preferably 5 days and especially preferably on 5 consecutive days, preferably in an amount per day of 50 mg/m$^2$ to 350 mg/m$^2$, more preferably 75 mg/m$^2$ to 250 mg/m$^2$, even more preferably 150 mg/m$^2$ to 250 mg/m$^2$, and especially about 200 mg/m$^2$; combined within said first week with the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), in a twice weekly administration schedule comprising about 500 mg or about 2000 mg (flat) of cyclo-(Arg-Gly-Asp-DPhe-NMeVal) per administration,

b) and in the directly following weeks 2, 3 and 4: the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), preferably as a single agent, in a twice weekly administration schedule comprising about 500 mg or about 2000 mg (flat) of cyclo-(Arg-Gly-Asp-DPhe-NMeVal) per administration.

Preferably, said method of treatment (D) is applied to the patient at least once, more preferably at least twice, even more preferably at least four times and especially six or more times, but generally less than 24 times, preferably less than 15 times, more preferably less than 11 times, for example 3 times, 4 times, 5 times, 6 times, 7 times or 12 times, preferably at least if it is administered without a pause, break or gap of at least one week, preferably of at least four weeks. After such a pause, gap or break, the above described administration scheme can be repeated once or several times, if necessary.

With respect to this method, said specific integrin ligand is more preferably administered in a timed administration as described herein, more preferably 1,5 to 20 hours (h), preferably 2 to 16 h, more preferably 2 to 12 h, even more preferably 2 to 10 h, even more preferably 3 to 10 h and especially 2 to 8 h prior to the application of the radiotherapy.

With respect to this method, the temozolomide is preferably administered orally, preferably With respect to this method, the temozolomide is preferably administered orally, preferably in analog manner as described herein for the timed administration of the specific integrin ligand, or preferably within 5 h, preferably within 3 h and especially within 1 h before or after, preferably after, the delivery of the radiotherapy.

The afore described method of treatment (D) can optionally be combined with one or more weeks of treatment, comprising the administration of the specific integrin ligand cyclo-(Arg-Gly-Asp-DPhe-NMeval) and/or the pharmaceutically acceptable salts thereof, preferably cyclo-(Arg-Gly-Asp-DPhe-NMeVal), to a patient, preferably as a single agent, in a twice weekly administration schedule comprising about 500 mg or about 2000 mg (flat) of cyclo-(Arg-Gly-Asp-OPhe-NMeVal) per administration, before, preferably directly before, and/or following, preferably directly following the afore described method of treatment (D). Preferably, the method of treatment (D) is combined with one or more weeks of additional treatment, comprising the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), to a patient, preferably as a single agent, in a twice weekly administration schedule comprising about 500 mg or about 2000 mg (flat) of cyclo-(Arg-Gly-Asp-DPhe-NMeVal) per administration, at least in the week directly prior to the first week of the method of treatment (D), and/or at least one week of said additional treatment (with or without pauses), for example untill progression, stable disease or healing occurs.

The afore described method of treatment (D) is especially advantageous in the treatment of tumors or cancer selected from astrocytoma, preferably astrocytoma grade II, III and/or IV, glioblastoma, preferably glioblastoma multiforme.

Preferred is a method of treatment (E),
comprising the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), to a patient, preferably as a single agent, in a twice weekly administration schedule comprising about 500 mg or about 2000 mg (flat) of cyclo-(Arg-Gly-Asp-DPhe-NMeVal) per administration, preferably for one week or for at least two consecutive weeks.

If the method of treatment (E) is applied for two or more consecutive weeks, it is preferably applied to the patient at least for four consecutive weeks, more preferably at least for six consecutive weeks, for example until tumor progression, stable disease or healing occurs. Preferably, the method of treatment (E) is combined with one or more of the above/below described methods of treatment (A), (B), (C) and/or (D). If the method of treatment (E) is combined with one or more of the above/below described methods of treatment (A), (B), (C) and/or (D), it is preferably applied in advance or at the end of said methods of treatment (A), (B), (C) and/or (D), or both in advance and at the end of said methods. Preferred combinations including one or more methods of treatment (A), (B), (C) and/or (D) and additionally the method of treatment (E) are given below.

Preferred is a method of treatment, wherein two or more of the treatment regimens, administration schedules and/or methods of treatment described herein are combined. More preferred is a method of treatment, comprising two or more of the above/below described methods of treatment (A), (B), (C), (D), (E) and/or (F), preferably including one or more of the preferred options as described with respect to the respective method (A), (B), (C), (D), (E) and/or (F). Especially preferred is a method of treatment, consisting of three or more of the above/below described methods of treatment (A), (B), (C), (D), (E) and/or (F), preferably including one or more of the preferred options as described with respect to the respective methods (A), (B), (C), (D), (E) and/or (F).

Preferred is a method of treatment (A), wherein said method is applied to the patient for 2 to 8 weeks and especially 6 weeks, preferably without a pause.

Preferred is a method of treatment, comprising or consisting of:

the method of treatment (E), wherein said method is applied to the patient for 1 to 4 weeks, preferably without a pause, directly followed by
the method of treatment (A), wherein said method is applied to the patient for 2 to 8 weeks and especially 6 weeks, preferably without a pause,
optionally directly followed by
the method of treatment (E), wherein said method is applied to the patient for at least for weeks, or preferably until progression, stable disease, partial/total response or healing occurs.

Preferred is a method of treatment, comprising or consisting of:

the method of treatment (E), wherein said method is applied to the patient for 1 to 4 weeks without a pause, more preferably about one week,
directly followed by
the method of treatment (B), wherein said method is applied to the patient for 2 to 8 weeks and especially 6 weeks, preferably without a pause,

optionally directly followed by
the method of treatment (E), wherein said method is applied to the patient for at least four weeks, or preferably until progression, stable disease, partial/total response or healing occurs.

Preferred is a method of treatment, comprising or consisting of:

the method of treatment (B), wherein said method is applied to the patient for 2 to 8 weeks and especially 6 weeks, preferably without a pause,
directly followed by
the method of treatment (D), wherein said method is applied to the patient 2 to 12 times, more preferably 4 to 8 times and especially about 6 times, preferably without a pause.

Preferred is a method of treatment, comprising or consisting of:

the method of treatment (B), wherein said method is applied to the patient for 2 to 8 weeks and especially 6 weeks, preferably without a pause,
optionally directly followed by
the method of treatment (E), wherein said method is applied to the patient for one or more weeks, preferably one week, directly followed by
the method of treatment (D), wherein said method is applied to the patient 2 to 12 times, more preferably 4 to 8 times and especially about 6 times, preferably without a pause.

Preferred is a method of treatment, comprising or consisting of:

the method of treatment (B), wherein said method is applied to the patient for 2 to 8 weeks and especially 6 weeks, preferably without a pause,
directly followed by
the method of treatment (E), wherein said method is applied to the patient for one or more weeks, preferably one week, directly followed by
the method of treatment (D), wherein said method is applied to the patient 2 to 12 times, more preferably 4 to 8 times and especially about 6 times, preferably without a pause.

Preferred is a method of treatment, comprising or consisting of:

the method of treatment (E), wherein said method is applied to the patient for 1 to 4 weeks without a pause, more preferably about one week,
directly followed by
the method of treatment (B), wherein said method is applied to the patient for 2 to 8 weeks and especially 6 weeks, preferably without a pause,
optionally directly followed by
the method of treatment (E), wherein said method is applied to the patient for one or more weeks, preferably one week, directly followed by
the method of treatment (D), wherein said method is applied to the patient 2 to 12 times, more preferably 4 to 8 times and especially about 6 times, preferably without a pause,
optionally directly followed by
the method of treatment (E), wherein said method is applied to the patient for at least 4 weeks, or preferably until progression, stable disease, partial/total response or healing occurs.

Preferred is a method of treatment, comprising or consisting of:

the method of treatment (E), wherein said method is applied to the patient for 1 to 4 weeks without a pause, more preferably about one week,
directly followed by
the method of treatment (B), wherein said method is applied to the patient for 2 to 8 weeks and especially 6 weeks, preferably without a pause,
directly followed by
the method of treatment (E), wherein said method is applied to the patient for one or more weeks, preferably one week, directly followed by

the method of treatment (D), wherein said method is applied to the patient 2 to 12 times, more preferably 4 to 8 times and especially about 6 times, preferably without a pause,
optionally directly followed by
the method of treatment (E), wherein said method is applied to the patient for at least 4 weeks, or preferably until progression, stable disease, partial/total response or healing occurs.

A preferred subject of the instant invention relates to a method or a use according the invention, wherein said specific integrin ligand is preferably administered on days on which the fractionated focal radiotherapy is administered or delivered to the patient, and preferably wherein the administration is a timed administration as described herein.

More preferably, the radiotherapy is administerd or delivered on one or more days on which the administration of the specific integrin ligand cyclo-(Arg-Gly-Asp-DPhe-NMeVal) and/or the pharmaceutically acceptable salts thereof, preferably cyclo-(Arg-Gly-Asp-DPhe-NMeVal), also takes place.

Preferred is thus is a method for treating astrocytoma wherein:

a) in week 1, optionally cyclo-(Arg-Gly-Asp-DPhe-NMeVal) is administered to the patient in a weekly administration scheme as described herein,
b) in weeks 2 - 7, at least one cancer cotherapeutic agent as described herein is applied to the patient, preferably selected from chemotherapeutic agents and radiotherapy as described herein, together with the administration of cyclo-(Arg-Gly-Asp-DPhe-NMeVal) in a weekly administration scheme as described herein,
c) in weeks 8 - 11, cyclo-(Arg-Gly-Asp-DPhe-NMeVal) is administered to the patient in a weekly administration scheme as described herein,
d) in weeks 12 - 35, cyclo-(Arg-Gly-Asp-DPhe-NMeVal) is administered to the patient in a weekly administration scheme as described herein, every fourth week supplemented by the administration of a cancer cotherapeutic agent other than radiotherapy, preferably the chemotherapeutic agent of step b), wherein the cancer cotherapeutic agent other than radiotherapy is preferably administered in the weeks 12, 16, 20, 24, 28 and 32.

A timed administration as described herein is preferred for cyclo-(Arg-Gly-Asp-DPhe-NMeVal) during weeks 2 - 7. The cancer cotherapeutic agent other than radiotherapy to be applied in steps b) and c) is selected from temozolomide.

Preferred is thus a method for treating astrocytoma wherein:

d) in week 1, optionally cyclo-(Arg-Gly-Asp-DPhe-NMeVal) is administered to the patient in a weekly administration scheme as described herein,
e) in weeks 2 - 7, radiotherapy as described herein, preferably fractionated or a focal radiotherapy as described herein, is applied to the patient, preferably, together with the administration of cyclo-(Arg-Gly-Asp-DPhe-NMeVal) in a weekly administration scheme as described herein, and together with at least one chemotherapeutic agent,
f) in weeks 8 - 11, cyclo-(Arg-Gly-Asp-DPhe-NMeVal) is administered to the patient in a weekly administration scheme as described herein,
e) in weeks 12 - 35, cyclo-(Arg-Gly-Asp-DPhe-NMeVal) is administered to the patient in a weekly administration scheme as described herein, every fourth week supplemented by the administration of the at least one chemotherapeutic agent of step b), wherein said chemotherapeutic agent is preferably administered in the weeks 12, 16, 20, 24, 28 and 32.

In this method of treatment, the at least one chemotherapeutic agent is selected from temozolomide . A timed administration as described herein is preferred for cyclo-(Arg-Gly-Asp-DPhe-NMeVal) during weeks 2 - 7.

Preferred is a method of treatment (F), comprising or preferably consisting of the following steps or treatments:

• Cyclo-(Arg-Gly-Asp-DPhe-NMeVal) is administered at a flat dose of about 500 mg or about 2000 mg by i. v. twice a week (preferably on Day 1 and 4, e. g., Monday and Thursday or Tuesday and Friday) for 35 weeks, preferably without pause. Treatment with cyclo-(Arg-Gly-Asp-DPhe-NMeVal) preferably begins on Day 1 of each week;

• Additional treatment (1) (Weeks 2 - 7 (max. of 7 weeks)):

Beginning on Day 1 of Week 2, treatment with TMZ and RT will be administered in addition to the cyclo-(Arg-Gly-Asp-DPhe-NMeVal), as follows:

Temomozolomide (TMZ) is administered orally for 6 weeks, at a daily dose of about 75 mg/m$^2$ (7 days a week),

Focal radiotherapy (RT) is delivered for 6 weeks, preferably once daily at about 2 Gy per fraction, 5 consecutive days/week, for a total of about 60 Gy. (preferably to be prescribed according to the guidelines of the International Commission on Radiological Units). Adequate immobilization masks can optionally be used to ensure reproducibility. The treatment volume can optionally be determined on the basis of preoperative Gd-MRI of the brain. Treatment volume preferably includes the contrast enhancing lesion as determined by the Gd-MRI, preferably plus plus a 2- to 3-cm margin around that lesion.

- Additional treatment (2) (Weeks 8 - 35)):

    Beginning 4 weeks after the end of RT (i.e. in Week 12), concomitant to cilengitide treatment, subjects receive TMZ chemotherapy at a dose of 150 - 200 mg/m$^2$ daily for 5 days (preferably days 1 to 5 of a given treatment week) every 4 weeks (i.e. Weeks 12, 16, 20, 24, 28 and 32) for up to 6 cycles.

In this method of treatment (F), the cyclo-(Arg-Gly-Asp-DPhe-NMeVal) is preferably administered in timed administration as described herein, preferably with respect to the administration of the RT and/or the TMZ; for example, the cyclo-(Arg-Gly-Asp-DPhe-NMeVal) is administered about 3 hours (h) (= start of the about 1 hour infusion) before the delivery of the RT; and TMZ is preferably after administration of the cyclo-(Arg-Gly-Asp-DPhe-NMeVal), but preferably before the delivery of the RT, more preferably within two hours before the delivery of the RT, even more preferably within about one hour before the delivery of the RT, and especially about one hour before the delivery of the RT. However, it can optionally also be suitable to administer the TMZ within about one hour after the delivery of the RT. However, the 35 week schedule of the method of treatment (F) can be terminated or shortened, if unacceptable adverse effects, an early progression, stable disease or healing of the patient occur.
A preferred method of treatment is depicted in the scheme below:

Cilengitide 500 mg i.v., 2x/week, until progression

Additional treatments:
TMZ 75 mg/m$^2$ p.o. daily for 6 weeks (during radiation),
Followed by 150–200 mg/m$^2$ p.o. day 1–5 every 28 days for 6 cycles (maintenance therapy)
Focal RT 5x/week – 30 x 200 cGy, total dose 60 Gy

Cilengitide (EMD121 974) and temozolomide with concomitant radiation therapy, followed by cilengitide and temozolomide maintenance therapy in subjects in need thereof, such as subjects with newly diagnosed glioblastoma
The methods of treatment as described herein and especially the methods of treatment (A) to (F) as described above, can optionally be followed by a continuing treatment comprising cyclo-(Arg-Gly-Asp-DPhe-NMeVal), preferably a continuing treatment as given below:
Subjects who complete 35 weeks of treatment without progression and preferably for whom continued treatment with cyclo-(Arg-Gly-Asp-DPhe-NMeVal) is considered beneficial, e. g. by the physician, may continue on cyclo-(Arg-Gly-Asp-DPhe-NMeVal) therapy (twice a week about 500 mg or about 2000 mg, i. v.). Accordingly, after the first 35 weeks of therapy, said cyclo-(Arg-Gly-Asp-DPhe-NMeVal) therapy can be continued until progression, stable disease, healing or unacceptable adverse effects occur.
Preferred are methods of treatment as described herein and preferably one or more of the methods of treatment, preferably selected from the group consisting of method of treatment (A), method of treatment (B), method of treatment (C), method of treatment (D), method of treatment (E), method of treatment (F), and combinations thereof, wherein the twice weekly administration scheme regarding cyclo-(Arg-Gly-Asp-DPhe-NMeVal) and/or the pharmaceutically acceptable dervatives, solvates and/or salts thereof, is substituted by a three times weekly administration scheme, preferably a three times weekly administration scheme as described herein, or a five times weekly administration scheme, preferably a five times weekly administration scheme as described herein. These preferred methods can preferably be advantageously applied to patients that do not belong to group of "methylated patients" according to the invention. The criteria for the "methylated patients" are given and discussed in detail below. Preferably, patients do not belong to group of "methylated patients" that can not be shown to have increased DNA methylation status, partial or complete methylation of at least one promotor of at least one MGMT gene and/or or only a Moderate MGMT protein level, or preferably less, in comparison to the MGMT protein level expressed by normal lymphocytes.

An especially preferred subject of the instant invention is the use of a specific integrin ligand selected from the group consisting of cyclo-(Arg-Gly-Asp-DPhe-NMeVal), the pharmaceutically acceptable solvates and salts thereof, and especially the use of cyclo-(Arg-Gly-Asp-DPhe-NMeVal) for the manufacture of a medicament to be used in the methods of treatment described herein, and especially for the manufacture of a medicament to be used in one or more of the methods of treatment, preferably selected from the group consisting of method of treatment (A), method of treatment (B), method of treatment (C), method of treatment (D), method of treatment (E), method of treatment (F), and combinations thereof.

If fractionated focal radiotherapy is applied with respect to brain metastases, preferably brain metastases of other cancer types as described herein, it preferably consists of about 25 to 45 Gy, more preferably 30 to 40 gy, preferably delivered in frations of 1.5 to 3.5, more preferably 1.8 to 3, e. g. about 2 Gy or about 3 Gy, preferably over a period of about three weeks, preferably 5 days a week.

Preferred is a method of prophylactic irradiation, preferably prophylactic cranial irradiation or prophylactic mediastinal irradiation, comprising administering at least one specific integrin ligand, more preferably at least one specific integrin ligand as described herein, comprising cyclo-(Arg-Gly-Asp-DPhe-NMe-Val) and especially preferably consisting of cyclo-(Arg-Gly-Asp-DPhe-NMeVal) and/or a pharmaceutically acceptable salt thereof, and radiotherapy, preferably fractionated focal radiotherapy as described herein.

In all of the above given methods of treatment or methods of prophylactic irradiation, is timed administration of the at least one specific integrin ligand is preferred.

With respect to the methods of treatment, administered amounts and/or the administration schemes described herein regarding of the specific integrin ligand cyclo-(Arg-Gly-Asp-DPhe-NMeVal) and/or a pharmaceutically acceptable salt thereof, preferably of cyclo-(Arg-Gly-Asp-DPhe-NMeVal), the amounts of (about) 500 mg or (about) 1000 mg to be administered at each administration as well as the amounts of (about) 1000 mg, (about) 1500 mg, (about) 2000 mg, (about) 2500 mg, (about) 4000 mg and (about) 6000 mg given for the weekly administration schemes are preferably calculated on the compound cyclo-(Arg-Gly-Asp-DPhe-NMeVal) as such (which is also referred to as the inner or internal salt of cyclo-(Arg-Gly-Asp-DPhe-NMeVal).

Accordingly, if a different form, such as the pharmacologically acceptable salts and solvates, of the specific integrin ligand cyclo-(Arg-Gly-Asp-DPhe-NMeVal) is to be administered to the patient, it is preferably administered in an amount equimolar to the amounts given above for the compound cyclo-(Arg-Gly-Asp-DPhe-NMeVal) as such.

The specific integrin ligand to be used according to the invention surprisingly show an advantageously improved effect on patients that are having increased DNA methylation status, are having a partial or complete methylation of at least one promoter of at least one MGMT gene and/or are having an abnormal level of MGMT protein, especially an abnormal low level of MGMT protein. Accordingly, the invention provides the use of medicaments that can be advantageously used to treat patients associated with one or more of the aforementioned effects or defects.

Therefore, subject of the instant invention is a medicament as described herein for use in the treatment of patients, wherein the medicament is to be used in the treatment of patients having an increased DNA methylation status, patients showing partial or complete methylation of at least one promotor of at least one MGMT gene and or patients having an abnormal level of MGMT protein, especially an abnormal low level of MGMT protein. Such patients are preferably referred to as "methylated patients".

These subjects are explained and discussed in more detail below:

Methylation of the DNA-repair gene $O^6$-methylguanine-DNA methyltransferase (MGMT), more correctly called $O^6$-methylguanine-DNA methyltransferase repair gene or short MGMT repair gene, causes gene silencing. This epigenetic modification has been associated with a favourable prognosis in patients with many different cancer types, such as glioblastoma (GBM), who receive alkylating agents, for example, nitrogen mustards, ethyleneimine compounds, alkyl sulphonates and other compounds with an alkylating action, preferably selected from nitrosoureas, preferably ACNU, BCNU and CCNU, busulfan, melphalan, carboplatin, cisplatin, oxaliplatin, cyclophosphamide, dacarbazine, carmustine, ifosfamide and lomustine, temozolomide and altretamine, or campthothecin. Accordingly, there is a relationship between MGMT promoter methylation and the survival rate and sensitivity to alkylating agents, such as temozolomide. The MGMT enzyme removes alkyl groups from the 06 position of guanine, the site of a number of chemotherapy-induced DNA alkylations. These chemotherapy induced alkylations lead to DNA damage in the tumor cells, including DNA double strand breaks and mismatches, which trigger apoptosis and cytotoxicity [5,6]. The MGMT enzyme repairs DNA damage, thus interfering with the therapeutic effects of chemotherapy alkylating agents [7-10]. Methylation of discrete regions of the MGMT promoter CpG island is associated with silencing of the gene and diminished DNA-repair enzyme activity [11-13]. Previous studies have indicated that 30-40% of GBM patients have methylated MGMT promoter [1-4].

The MGMT promoter methylation and thus the methylation status of the MGMT can be advantageously determined using a 2-stage methylation specific PCR analysis on DNA extracted from tumor specimens, such as tumour specimens which have been snap frozen at surgery. The Methylation specific PCR analysis can be easily performed according to methods in the art. Preferably it can be performed by the Method by Hegi et al., NEJM, 2005, 352; 997-1003); the following method has been successfully been used in a Phase III trial assessing the methylation status of a subset of the patients (tissue available):

**DNA extraction and methylation-specific polymerase chain reaction**

Genomic DNA is isolated from one or two paraffin sections of glioblastoma tissue (Ex-Wax DNA Extraction Kit S4530, Chemicon) (proteinase digestion lasted a maximum of six hours). DNA is denatured with sodium hydroxide in a volume of 35 $\mu$l and subjected to bisulfite treatment in a volume of 360 $\mu$l (4.4 M sodium bisulfite and 20 mM hydroquinone) for five hours at 55°C and then purified (Wizard DNA Clean-Up System A7280, Promega).
Unmethylated cytosine, but not its methylated counterpart, is modified into uracil by the treatment. The methylation-specific polymerase chain reaction (PCR) is performed in a two-step approach. [Palmisano WA, Divine KK, Saccomanno G, et al. Predicting lung cancer by detecting aberrant promoter methylation in sputum. Cancer Res 2000;60:5954-8.]
The results can be confirmed in an independent experiment, starting with reisolation of DNA from the tumor. The PCR products are separated on 4 percent agarose gels. The investigators who selected and analyzed the glioblastoma samples are blinded to all clinical information.
Alternatively, it can be performed according to the method described by Donson et al. in Journal Pedriatic Blood Cancer, 2006.
According to Donson et al., the MGMT promoter methylation/methylation status of the MGMT can be advantageously determined according to the following procedure:

DNA Extraction and Methylation-Specific Polymerase Chain Reaction

Genomic DNA is isolated from snap frozen tumor obtained at surgery (COMIRB 95-500) and GBM cell-lines using a DNeasy kit (Qiagen, Valencia, CA). DNA methylation patterns in the CpG island of the MGMT gene are determined by methylation specific PCR. This procedure involves chemical modification of unmethylated, but not methylated cytosines to uracil, followed by a nested, twostage PCR [17]. One microgram of DNA is denatured with sodium hydroxide (final conc. 0.3 M) in a volume of 55 ml and subjected to bisulfite treatment in a volume of 610 ml (3.3 M sodium bisulfite and 0.5mMhydroquinone) for 16 hr at 558C and then purified using the Wizard DNA Clean-Up System (Promega, Madison, WI). PCR is performed to amplify a 289-bp fragment of the MGMT gene including a portion of the CpG-rich promoter region. The primers recognize the bisulfite-modfied template but do not discriminate between methylated and unmethylated alleles. Primer sequences used in the stage 1 amplification of theMGMTgene are as follows: MGMT-stage 1-Forward, 50-GGATATGTTGGGATAGTT- 30; and MGMT-stage 1-Reverse, 50-CCAAAAACCCCAAACCC- 30. Master Mix (Fermentas, Hanover, MD). The PCR amplification protocol for stage 1 is as follows: 958C for 10 min, then denature at 958C for 30 sec, anneal at 528C for 30 sec, extension at 728C for 30 sec for 40 cycles followed by a 10min final extension.A25-ml volume is used in all of the PCR reactions. The stage-1 PCR products are diluted 50-fold, and 5 ml of this dilution is subjected to a stage-2 PCR in which primers specific to methylated or unmethylated template are used. Primer sequences for the stage 2 PCR for the unmethylated reaction are MGMT-stage 2-Forward, 50-TTTGTGTTTTGATGTTTGTAGGTTTTTGT-30 and MGMT-stage 2-Reverse, 50-AACTCCACACTCTTCCAAAAACAAAACA- 30 and for the methylated reaction MGMT-stage 2-forward 50-TTTCGACGTTCGTAGGTTTTCGC- 30 and MGMT-stage 2-reverse 50-GCACTCTTCCGAAAACGAAACG- 30. The PCR amplification protocol for stage 2 is as follows: 958C for 10 min, then denature at 958C for 15 sec, anneal at 628C for 15 sec, extension at 728C for 15 sec for 40 cycles followed by a 10 min final 728C extension. DNA from normal human lymphocytes treated in vitro with SssI methyltransferase (New England Biolabs, Beverly, MA) is used as positive control for methylated alleles of MGMT and untreated DNA from normal lymphocytes is used as negative control for methylated alleles of MGMT. Each PCR reaction (10 ml) is directly loaded onto 4% agarose gel, stained with ethidium bromide and visualized under UV illumination. Statistical Analysis can be performed with methods known in the art, such as the methods by Kaplan-Meier, correlation and statistical significance analyses, for example using the Prism statistical analysisprogram (GraphPad Software, Inc., San Diego, CA). Methylguanine-DNA methyltransferase promoter methylation status analysis is performed on snap frozen tissue of the patients. MGMT methylation status can regularly be determined out the tumors. In a part of the patients, the samples tested for MGMT promoter methylation status proved to be partially methylated (Fig. A). None of the samples showed complete methylation. The incomplete methylation observed may be due to tumor heterogeneity, infiltrating peripheral blood lymphocytes and/or vasculature. For comparison purposes, it can be determined whether partial methylation of the tumor MGMT promoter can be responsible for this observation by investigating the MGMT promoter methylation status of 6 GBM cell-lines, including cell-line 145 which is established from a patient who is treated with temozolomide and who's snap frozen tumor is also analyzed in the above study. In four out of the six celllines studied, partial methylation of promoter is observed (Fig. B). The results show that even in pure GBM cell-lines, partial MGMT promoter methylation can exist.

## Fig. A

Fig. A. Methylation status of the MGMT promoter in GBM biopsy specimens, as determined by a nested methylation-specific PCR assay. DNA from normal peripheral blood lymphocytes (PBL) is used as a control for the unmethylated MGMT promoter (U), enzymatically methylated DNA from PBL (MPBL) served as a positive control for the methylated MGMT promoter (M), and water is used as a negative control for the PCR. A 100-bp marker ladder is loaded to estimate molecular size, as shown on the left scale (L).

## Fig. B

Fig. B. Methylation status of the MGMTpromoter inGBMcell-lines, as determined by a nested methylation-specific PCR assay. A 100-bp marker ladder is loaded to estimate molecular size, as shown on the left scale (L).

The MGMT analysis technique described above has been employed in the majority of recent studies showing MGMT methylation to be a successful predictor of response to alkylating agents [1-3]. This technique has superseded earlier techniques of enzyme activity measurement after it was demonstrated that MGMT methylation was the main cause of loss of MGMT enzymatic activity in GBM.

Patients that are tested as patients showing MGMT methylation or that can be tested as patients showing MGMT methylation, preferably using the above described method, an analog method thereof, or any other method which is equally suitable according to the understanding of the ones skilled in the art, are to be regarded as "methylated patients" according to the invention, more preferably as patients having an increased DNA methylation status and/or as patients showing partial or complete methylation of at least one promotor of at least one MGMT gene. They thus belong to the collective of patients that can be especially advantageously treated by the methods of treatment or the medicaments according to the invention.

However, such techniques, e.g. the method described below, can preferably be used in concordance with the instant invention with respect to the MGMT status.

Chemotherapeutic efficacy, the ability of chemotherapy to eradicate tumor cells without causing lethal host toxicity, depends on drug selectivity. One class of anticancer drugs, alkylating agents, cause cell death by binding to DNA which structurally distorts the DNA helical structure preventing DNA transcription and translation. In normal cells, the damaging action of alkylating agents can be repaired by cellular DNA repair enzymes, in particular $O^6$-methylguanine-DNA methyltransferase (MGMT) also known as $O^6$-alkylguanine-DNA-alkyltransferase (AGAT). The level of MGMT varies in tumor cells, even among tumors of the same type. The gene encoding MGMT is not commonly mutated or deleted. Rather, low levels of MGMT in tumor cells are due to an epigenetic modification; the MGMT promoter region is methylated, thus inhibiting transcription of the MGMT gene and preventing expression of MGMT. Methylation has been shown by several lines of evidence to play a role in gene expression, cell differentiation, tumorigenesis, X-chromosome inactivation, genomic imprinting and other major biological processes. In eukaryotic cells, methylation of cytosine residues that are immediately 5' to a guanosine, occurs predominantly in cytosine-guanine (CG) poor regions. In contrast, CpG islands remain unmethylated in normal cells, except during X-chromosome inactivation and parental specific imprinting where methylation of 5' regulatory regions can lead to transcriptional repression. Expression of a tumor suppressor gene can also be abolished by de novo DNA methylation of a normally unmethylated CpG. Hypermethylation of genes encoding DNA repair enzymes can serve as markers for predicting the clinical response to certain cancer treatments. Certain chemotherapeutic agents (including alkylating agents for example) inhibit cellular proliferation by cross-linking DNA, resulting in cell death.

Treatment efforts with such agents can be thwarted and resistance to such agents develops because DNA repair enzymes remove the cross-linked structures. In view of the deleterious side effects of most chemotherapeutic drugs, and the ineffectiveness of certain drugs for various treatments, it is desirable to predict the clinical response to treatment with chemotherapeutic agents.

U.S. Pat. No. 6,773,897 discloses methods relating to chemotherapeutic treatment of a cell proliferative disorder. In particular, a method is provided for "predicting the clinical response to certain types of chemotherapeutic agents", including specific alkylating agents. The method entails determination and comparison of the methylation state of nucleic acid encoding a DNA repair enzyme from a patient in need of treatment with that of a subject not in need of treatment. Any difference is deemed "predictive" of response. The method, however, offers no suggestion of how to improve clinical outcome for any patient with an unfavorable "prediction". Temozolomide is an alkylating agent available from Schering Corp. under the trade name of Temodar® in the United States and Temodal® in Europe. Temodar® Capsules for oral administration contain temozolomide, an imidazotetrazine derivative. The chemical name of temozolomide is 3,4-dihydro-3-methyl-4-oxoimidazo[5,1-d]-as-tetrazine-8-carboxamide (see U. S. Pat. No. 5,260,291). The cytotoxicity of temozolomide or metabolite of it, MTIC, is thought to be primarily due to alkylation of DNA. Alkylation (methylation) occurs mainly at the $O^6$ and $N^7$ positions of guanine. Temodar® (temozolomide) Capsules are currently indicated in the United States for the treatment of adult patients with newly diagnosed gliobastoma multiforme as well as refractory anaplastic astrocytoma, i.e. patients at first relapse who have experienced disease progression on a drug regimen containing a nitrosourea and procarbazine. Temodal® is currently approved in Europe for the treatment of patients with malignant glioma, such as glioblastoma multiforme or anaplastic astrocytoma showing recurrence or progression after standard therapy.

According to the invention, alternatively to the method described above, the level of methylation of MGMT gene is assessed by determining the level of MGMT protein in a sample obtained from the patient. The level can be classified as being "Very Low" "Low", "Moderate", or "High", preferably as described in more detail below.

Assessing whether or not the MGMT gene is methylated can be performed using any method known to one skilled in the art. Techniques useful for detecting methylation of a gene or nucleic acid include, but are not limited to those described by Ahrendt et aL, J. Natl. Cancer Inst., 91:332-339 (1999); Belsinky etal., Proc. Natl. Acad. Sci. U.S.A., 95:11891-11896 (1998), Clark et al., NucleicAcids Res., 22:2990-2997 (1994); Herman etaL, Proc Natl Acad Sd U.S.A., 93:9821-9826 (1996); Xiong and Laird, Nucleic Acids Res., 25:2532-2534 (1997); Eads et aL, Nuc. Acids. Res., 28:e32 (2002); Cottrell et al., Nucleic Acids Res., 32:1-8 (2004). All references cited herein are incorporated herein by reference.

Methylation-specific PCR (MSP; Herman et al., Proc. Natl. Acad Sci. USA, 93(18):9821-9826 (1996); Esteller etal., Cancer Res., 59:793-797 (1999)) see also U.S. Pat. No. 5,786,146, issued Jul. 28, 1998; U.S. Pat. No. 6, 017,704, issued Jan. 25, 2000; U.S. Pat. No. 6,200,756, issued Mar. 13, 2001; and U.S. Pat. No. 6,265,171, issued Jul. 24, 2001; U.S. Pat. No. 6, 773,897 issued August 10, 2004; the entire contents of each of which is incorporated herein by reference can rapidly assess the methylation status of virtually any group of CpG sites within a CpG island, independent of the use of methylation-sensitive restriction enzymes. This assay entails initial modification of DNA by sodium bisulfite, converting all unmethylated, but not methylated, cytosines to uracil, and subsequent amplification with primers specific for methylated versus unmethylated DNA. MSP requires only small quantities of DNA, is sensitive to 0.1% methylated alleles of a given CpG island locus, and can be performed on DNA extracted from paraffin-embedded samples. MSP eliminates the false positive results inherent to previous PCR-based approaches which relied on differential restriction enzyme cleavage to distinguish methylated from unmethylated DNA. This method is very simple and can be used on small amounts of tissue or a few cells.

An illustrative example of a Western blot assay useful for this embodiment of the invention to measure the level of MGMT protein in patient samples is presented in U.S. Pat. No. 5,817,514 by Li et al., the entire disclosure of which is incorporated herein by reference. Li et al. described monoclonal antibodies able to specifically bind either to native human MGMT protein or to human MGMT protein having an active site which is alkylated. An illustrative example of an immunohistochemical technique useful for this embodiment of the invention to measure the level of MGMT protein in patient samples is presented in U.S. Pat. No. 5, 407,804, the entire disclosure of which is incorporated herein by reference. Monoclonal antibodies are disclosed which are able to specifically bind to the MGMT protein in single cell preparations (immunohistochemical staining assays) and in cell-extracts (immunoassays).

The use of fluorescent read out coupled with digitization of the cell image is described and allows for quantitative measurement of MGMT levels in patient and control samples, including but not limited to tumor biopsy samples. Useful techniques for measuring the enzymatic acitivity of MGMT protein include but are not limited to methods described by: Mymes et al., Carcinogenesis, 5:1061-1 064 (1984); Futscher etal., Cancer Comm., 1: 65-73 (1989); Kreklaw etal., J. Pharmacol. Exper. Ther., 297(2):524-530 (2001); and Nagel et al., Anal. Biochem., 321(1):38-43 (2003), the entire disclosures of which are incorporated herein in their entireties.

According to one mode of this invention, the level of MGMT protein expressed by cells of the patient is assessed by measurement of the MGMT protein, e.g., by Western blot using an antibody specific to MGMT, see for example, U.S. Pat. No. 5,817,514 (supra) by Li et al. for a description of a Western blot assay to determine MGMT level. The level is compared to that expressed by normal lymphocytes known to express MGMT.

Patient MGMT protein levels are preferably classified as follows: Very Low = 0-30% of the MGMT expressed by normal lymphocytes; Low = 31-70% of the MGMT expressed by normal lymphocytes; Moderate = 71-90% and High = 91-300% or higher of the MGMT expressed by normal lymphocytes.

Patients that are tested as patients having Moderate or less MGMT protein levels or that can be tested as patients having Moderate or less MGMT protein levels, preferably using the above described method, an analog method thereof, or any other method which is equally suitable according to the understanding of the ones skilled in the art in the art, are to be regarded as "methylated patients" according to the invention. They thus belong to the collective of patients that can be especially advantageously treated by the methods of treatment or the medicaments according to the invention.

Accordingly, patients that have or can be shown to have Moderate (= 71-90%), preferably (Low = 31-70%) and more preferably Very Low (= 0-30%), of the MGMT expressed by normal lymphocytes are preferably to be regarded as "methylated patients" according to the invention, more preferably as patients having an increased DNA methylation status and/or as patients showing partial or complete methylation of at least one promotor of at least one MGMT gene. They thus belong to the collective of patients that can be especially advantageously treated by the methods of treatment or the medicaments according to the invention.

Thus, an especially preferred subject of the invention is a use as described herein, wherein the medicament is to be used in the treatment of patients having an increased DNA methylation status.

Thus, an especially preferred subject of the invention is a use as described herein, wherein the medicament is to be used in the treatment of patients showing partial or complete methylation of at least one promotor of at least one MGMT gene.

Thus, an especially preferred subject of the invention is a use as described herein, wherein the medicament is to be used in the treatment of patients, having a Moderate, preferably a Low and more preferably a Very Low level of MGMT protein, preferably in comparison of the MGMT expressed by normal lymphocytes.

Thus, an especially preferred subject of the invention is a use as described herein, wherein the medicament is to be used in the treatment of patients having an increased DNA methylation status, and wherein said method comprises the administration of one or more alkylating agents, preferably selected from, nitrogen mustards, ethyleneimine compounds, alkyl sulphonates and other compounds with an alkylating action, preferably selected from nitrosoureas, preferably ACNU, BCNU and CCNU, busulfan, melphalan, carboplatin, cisplatin, oxaliplatin, cyclophosphamide, dacarbazine, carmustine, ifosfamide and lomustine, temozolomide and altretamine, or campthothecin.

Thus, an especially preferred subject of the invention is a use as described herein, wherein the medicament is to be used in the treatment of patients showing partial or complete methylation of at least one promotor of at least one MGMT gene and wherein said method comprises the administration of one or more alkylating agents, preferably selected from, nitrogen mustards, ethyleneimine compounds, alkyl sulphonates and other compounds with an alkylating action, preferably selected from nitrosoureas, preferably ACNU, BCNU and CCNU, busulfan, melphalan, carboplatin, cisplatin, oxaliplatin, cyclophosphamide, dacarbazine, carmustine, ifosfamide and lomustine, temozolomide and altretamine, or campthothecin.

Thus, an especially preferred subject of the invention is a use as described herein, wherein the medicament is to be used in the treatment of patients, having a Moderate, preferably a Low and more preferably a Very Low level of MGMT protein, preferably in comparison of the MGMT expressed by normal lymphocytes, and wherein said method comprises the administration of one or more alkylating agents, preferably selected from, nitrogen mustards, ethyleneimine compounds, alkyl sulphonates and other compounds with an alkylating action, preferably selected from nitrosoureas, preferably ACNU, BCNU and CCNU, busulfan, melphalan, carboplatin, cisplatin, oxaliplatin, cyclophosphamide, dacarbazine, carmustine, ifosfamide and lomustine, temozolomide and altretamine, or campthothecin.

Methods to assess an increased DNA methylation status and/or showing partial or complete methylation of at least one promotor of at least one MGMT gene in patients are known in the art. Accordingly, patients to be advantagously treatable by a uses as described herein can readily determined by the ones skilled in the art.

A preferred subject of the instant invention is a use as described herein, wherein the medicament is to be used in the treatment of recurrent astrocytoma, for example in a second line or subsequent treatment setting.

A more preferred subject of the instant invention is a use as described herein, wherein the medicament is to be used in the treatment of recurrent cancer, for example in a second line or subsequent treatment setting, wherein the cancer is selected from the group consisting of astrocytoma, more preferably astrocytoma grade II, III and/or IV, and especially consisting of glioblastoma or glioblastoma multiforme.

An even more preferred subject of the instant invention is a use as described herein, wherein the medicament is to be used in the treatment of newly diagnosed astrocytoma, preferably in a first line treatment setting.

An especially preferred subject of the instant invention is a use as described herein, wherein the medicament is to be used in the treatment of newly diagnosed cancer, preferably in a first line treatment setting, wherein the cancer is selected from the group consisting of astrocytoma, more preferably astrocytoma grade II, III and/or IV, and especially consisting of glioblastoma or glioblastoma multiforme.

**Examples**

The following examples are given in order to assist the skilled artisan to better understand the present invention by way of exemplification. The examples are not intended to limit the scope of protection conferred by the claims. The features, properties and advantages exemplified for the compounds and uses defined in the examples may be assigned to other compounds and uses not specifically described and/or defined in the examples, but falling under the scope of what is defined in the claims.

**Example 1 [for technical information only]: Rat orthotopic glioblastoma model radiotherapy, Cilengitide (= cyclo-(Arg-Gly-Asp-DPhe-NMe-Val)) scheduling experiments**

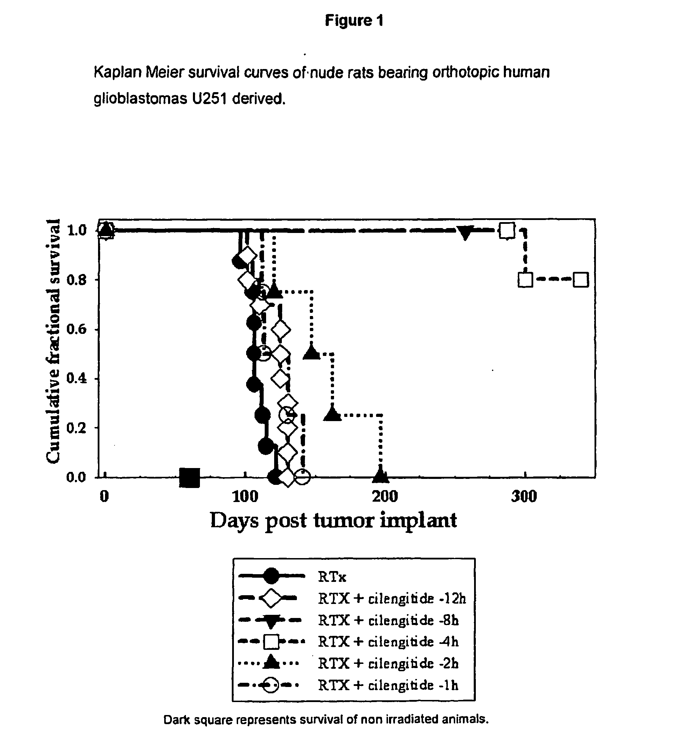

*NIH rnu* **nude rats are anaesthetized, restrained, and injected intracerebrally** 1 mm retro orbitally, 3mm to the right of the bregma and at a depth of 2.5 mm with 5x10E5 U251 human glioblastoma cells suspended in 10 ul of culture medium, using a #2701 Hamilton syringe fitted with a 26 gauge needle, essentially as previously described (Engebraaten et al., 1999). After 14 days, cilengitide (4 mg/kg) is given as an intraperitoneal bolus in PBS, at various time (8h, 4h, 2h, 1 h) prior to a single treatment with single, collimated, dorsal-ventral beam of 6 MV x-rays, so that 95-100% of the central axis dose of 25 Gy hit the tumor volume (Kim et al., 1999). Each of the 7 subsequent days the animals also received an identical i.p. bolus of cilengitide. The animals are maintained under ad libitum food and drink until they become moribund, or are sampled for tissue analysis (in the t-4 and t-8 h groups, where the animals are healthy past 230 days post tumor injection). A Kaplan-Meier survival curve is calculated and plotted (Fig.1) from the raw data (Table 1). All animals in the RT monotherapy group died by 120 d.

Reference List:

Engebraaten,O., Hjortland,G.O., Hirschberg,H., and Fodstad,O. (1999). Growth of precultured human glioma specimens in nude rat brain. J. Neurosurg. 90, 125-132.
Kim,J.H., Khil,M.S., Kolozsvary,A., Gutierrez,J.A., and Brown,S.L. (1999). Fractionated radiosurgery for 9L gliosarcoma in the rat brain. Int. J. Radiat. Oncol. Biol. Phys. 45, 1035-1040.

The Results are given in Table 1 below and Fig. 1:

**Table 1**

| 400,000 U251n Cells Inj. | | | | EMD Survival Study | | | |
|---|---|---|---|---|---|---|---|
| | | | | | | | **Days Post** |
| **Group** | **Time Pre-Irradiation** | **Animal#** | **Trtmnt** | **Date of Injection** | **Date of Radiation** | **Date of Termination** | **Implant** |
| | | | | | | | |
| 89 | 8 hours | G89-1 | Rt | 03.03.2005 | 17.03.2005 | (Sick) 6/7/2005 | 96 |
| 89 | 8 hours | G89-2 | Rt | 03.03.2005 | 17.03.2005 | (Sick) 6/17/2005 | 106 |
| 89 | 8 hours | G89-3 | Rt + EMD | 03.03.2005 | 17.03.2005 | (Healthy) 11/15/2005 | 257 |
| 89 | 8 hours | G89-4 | Rt + EMD | 03.03.2005 | 17.03.2005 | (Healthy) 11/15/2005 | 257 |

(continued)

| 400,000 U251n Cells Inj. | | | | EMD Survival Study | | | |
|---|---|---|---|---|---|---|---|
| Group | Time Pre-Irradiation | Animal# | Trtmnt | Date of Injection | Date of Radiation | Date of Termination | Days Post Implant |
| 89 | 8 hours | G89-5 | Rt + EMD | 03.03.2005 | 17.03.2005 | (Alive) 12/15/2005 | 287 |
| 89 | 8 hours | G89-6 | Rt + EMD | 03.03.2005 | 17.03.2005 | (Alive) 12/15/2005 | 287 |
| | | | | | | | |
| 90 | 4 hours | G90-1 | Rt | 05.04.2005 | 19.04.2005 | (Sick) 7/20/2005 | 106 |
| 90 | 4 hours | G90-2 | Rt | 05.04.2005 | 19.04.2005 | (Sick) 7/29/2005 | 115 |
| 90 | 4 hours | G90-3 | Rt + EMD | 05.04.2005 | 19.04.2005 | (Healthy) 11/29/2005 | 238 |
| 90 | 4 hours | G90-4 | Rt + EMD | 05.04.2005 | 19.04.2005 | (Healthy) 11/29/2005 | 238 |
| 90 | 4 hours | G90-5 | Rt + EMD | 05.04.2005 | 19.04.2005 | (Alive) 12/15/2005 | 254 |
| 90 | 4 hours | G90-6 | Rt + EMD | 05.04.2005 | 19.04.2005 | (Alive) 12/15/2005 | 254 |
| | | | | | | | |
| 91 | 2 hours | G91-1 | Rt | 12.04.2005 | 26.04.2005 | (Sick) 7/26/2005 | 105 |
| 91 | 2 hours | G91-2 | Rt | 12.04.2005 | 26.04.2005 | (Sick) 8/12/2005 | 122 |
| 91 | 2 hours | G91-3 | Rt + EMD | 12.04.2005 | 26.04.2005 | (Sick) 8/12/2005 | 120 |
| 91 | 2 hours | G91-4 | Rt + EMD | 12.04.2005 | 26.04.2005 | (Sick) 9/6/2005 | 147 |
| 91 | 2 hours | G91-5 | Rt + EMD | 12.04.2005 | 26.04.2005 | (Sick) 9/21/2005 | 162 |
| 91 | 2 hours | G91-6 | Rt + EMD | 12.04.2005 | 26.04.2005 | (Sick) 10/25/2005 | 196 |
| | | | | | | | |
| 92 | 1 hour | G92-1 | Rt | 12.05.2005 | 26.05.2005 | (Sick) 8/26/2005 | 106 |
| 92 | 1 hour | G92-2 | Rt | 12.05.2005 | 26.05.2005 | (Sick) 9/1/2005 | 112 |
| 92 | 1 hour | G92-3 | Rt + EMD | 12.05.2005 | 26.05.2005 | (Sick) 9/1/2005 | 112 |
| 92 | 1 hour | G92-4 | Rt + EMD | 12.05.2005 | 26.05.2005 | (Sick) 9/2/2005 | 113 |

(continued)

| 400,000 U251n Cells Inj. | | | | EMD Survival Study | | | |
|---|---|---|---|---|---|---|---|
| | | | | | | | Days Post Implant |
| Group | Time Pre-Irradiation | Animal# | Trtmnt | Date of Injection | Date of Radiation | Date of Termination | Implant |
| 92 | 1 hour | G92-5 | Rt + EMD | 12.05.2005 | 26.05.2005 | (Sick) 9/19/2005 | 130 |
| 92 | 1 hour | G92-6 | Rt + EMD | 12.05.2005 | 26.05.2005 | (Sick) 9/30/2005 | 141 |

Sick = moribund and removed from study
Healthy = indicates sampled for tissue at date shown, but alive at this point
Alive = surviving at time point shown.
Time pre-irradiation = when cilengitide 4 mg/kg is given.
Rt = radiotherapy 25 Gy
EMD = cilengitide bolus 4 mg/kg

American date convention in date of termination column, European in date of radiation column

**Example 2 [for technical information only]: Phase IIa Trial of Cilengitide ((= cyclo-(Arg-Gly-Asp-DPhe-NMe-Val))) Single Agent Therapy in Patients with Recurrent Glioblastoma**

Background: The present phase IIa study was designed to evaluate the safety, toxicity, and clinical activity of the cyclic RGD pentapeptide cilengitide((= cyclo-(Arg-Gly-Asp-DPhe-NMe-Val)), an inhibitor of integrins $av\beta3$ and $av\beta5$, as a single agent at doses of 500 and 2000 mg in patients (pts) with recurrent glioblastoma (GBM).

**Methods:** In this multicenter, open-label, randomized, uncontrolled study, pts with GBM and measurable disease that had relapsed after previous therapy with temozolomide and radiotherapy were randomized to receive cilengitide at doses of either 500 mg or 2000 mg i.v., 2x/week, until progression. Histopathology diagnosis and MRI imaging were subject to independent blinded review. The primary endpoint was Progression Free Survival (PFS) at 6 months (mths). Secondary endpoints included response, survival, time to disease progression, safety, tolerability and pharmacokinetics.

**Results:** Actual accrual; 81 pts (median Karnofsky Performance Status 80%; median age 57 yrs) at 15 sites. 41 pts received 500 mg and 40 pts to receive 2000 mg of i.v. cilengitide 2x/week. No obvious imbalance in prognostic factors was observed. Median infusions;16 [range, 4-179]. Treatment related NCI CTC grade 3 adverse events (AEs) included elevated liver enzymes (at 500 mg), arthralgia/ myalgia (at 500 mg), and weight increase/ edema (at 2000 mg) in 1 patient, respectively. No grade 4 therapy related AEs were reported by the investigators. One CTC grade 2 cerebral hemorrhage was reported, possibly related either to the drug or to the disease. The PFS rate at 6 mths was 16.1% (n=13/81 pts). 10 of these pts (12.3 %, n=4 with 500 mg, n=6 with 2000 mg) received 12 or more cycles of therapy (1 cycle=4 weeks). Six pts (7.4%) were still progression-free and on treatment at the time when this abstract was issued. In the 500 mg arm, median Overall Survival (mOS) was 6.5 mths [95% CI: 5.2-9.3 mths], 12 mth overall survival (OS) rate was 24.4%. In the 2000 mg arm, mOS was 9.9 mths [95% CI, 6.3-15.7 mths], 12 mth OS rate was 37.5%. Although not statistically significant, there was a trend towards better tumor control in pts receiving 2000 mg 2x/week.

**Conclusion:** Cilengitide was tolerated well in single agent therapy at two dose levels. Cilengitide demonstrated advantagous single agent activity in recurrent glioblastoma, with long term disease stabilisation in a subset of pts.

**Example 3: Phase I/IIa Trial of Cilengitide (= cyclo-(Arg-Gly-Asp-DPhe-NMe-Val)) and Temozolomide with Concomitant Radiotherapy, Followed by Temozolomide and Cilengitide Maintenance Therapy in Patients With Newly Diagnosed Glioblastoma (GBM).**

**Purpose:** To evaluate safety, toxicity, and efficacy of the combination of the cyclic RGD pentapeptide Cilengitide (= cyclo-(Arg-Gly-Asp-DPhe-NMe-Val)), an inhibitor of integrins $av\beta3$ and $av\beta5$, in addition to standard temozolomide (TMZ) and radiotherapy (RT).

**Patients and methods:** Fifty-two pts (PS 0-1: 92%, 2: 8%; median age 57 yrs) after biopsy (n= 9/17%) or tumor resection (n=43/83%) were treated with standard TMZ/RT (Stupp et al. NEJM 2005). In addition Cilengitide (500 mg i.v., 2x/week) was started one week before TMZ/RT and given throughout for the duration of chemotherapy or until progression. Primary endpoint was progression free survival rate at 6 months (target: 65%). Patients were followed with MRI every 2 months. Histopathologic diagnosis and MRI imaging were independently reviewed, MGMT promotor methylation status was assessed in 45 (86.5%) pts.

**Results:** Forty-six pts (92%) completed RT, $\geq$ 90% of concomitant TMZ was received by 42 pts and cilengitide by 45 pts. 20 pts (3 ongoing) completed 6 cycles of maintenance TMZ and cilengitide. Observed haematological grade 3 and 4 toxicity was: lymphopenia (28/52, 53.8%), thrombocytopenia (7/52 pt. 13.4%) and neutropenia (5/52, 9.6%). Treatment related non-hematologic grade 3 toxicities were reported for n=3/52 (5.7%) patients: constitutional symptoms (asthenia, fatigue, anorexia, n=3); elevated liver function tests (n=1), deep venous thrombosis and pulmonary embolism (n=1). One patient with a history of sigmoid diverticulosis experienced sigmoid perforation (grade 2). In total, 34/52 (65.4% [95% CI, 50.9-78.0%]) of the patients were progression free at 6 months. Pts with $O^6$-Methylguanine-DNA methyltransferase (MGMT) gene-promotor methylation in the tumor were more likely to reach 6 months PFS endpoint. In total, 34/52 (65.4% [95% CI, 50.9-78.0%]) of the pts were progression free at 6 months. A major contribution to the overall result was provided by a subgroup of patients (23/52 subjects, with methylated MGMT promoter, silencing the DNA repair enzyme MGMT), which showed a strong increase of the PFS-6 rate compared to historical control (91% vs. 69%). The other major subgroup (22/52, unmethylated MGMT promotor) showed a less relevant difference to the historical control (40.9% vs. 40%), which is likely to be significantly improved by a higher dosing of Cilengitide in comparison to the subgroup with methylated MGMT promoter. Overall the study reached its primary endpoint (PFS-6 = 65.4%)

Conclusion: The study reached its primary endpoint. The combination of the integrin inhibitor RGD peptide Cilengitide and TMZ/RT was well tolerated, PFS at 6 months is very advantagous. MGMT gene promotor methylation provides for even better prognosis.

## Claims

1. Use of at least one specific integrin ligand, selected from cyclo-(Arg-Gly-Asp-DPhe-NMeVal) and the pharmaceutically acceptable solvates and salts thereof,
   for the manufacture of a medicament for the treatment of astrocytoma, wherein the medicament is to be used in combination with temozolomide and radiotherapy.

2. Use according to claim 1, wherein the astrocytoma is glioblastoma or glioblastoma multiforme.

3. Use according to claim 1 and/or 2, wherein the radiotherapy is fractionated external beam radiation.

4. Use according to one or more of claims 1 to 3, wherein said specific integrin ligand is to be applied substantially concurrently or sequentially to the temozolomide and/or the radiotherapy.

5. Use according to one or more of claims 1 to 4, wherein the at least one integrin ligand is cyclo-(Arg-Gly-Asp-DPhe-NMeVal).

6. Use according to one or more of claims 1 to 5, wherein at least one of the specific integrin ligands is to be administered 1 to 8 hours (h) prior to the application of the radiotherapy and/or the temozolomide.

7. Use according to claim to one or more of claims 1 to 6, wherein at least one of the specific integrin ligands is to be administered 2 to 6 hours prior to the application of the radiotherapy and/or the temozolomide.

8. Use according to claim 1, wherein at least one specific integrin ligand, comprising cyclo-(Arg-Gly-Asp-DPhe-NMe-Val), the pharmaceutically acceptable solvates and/or salts thereof, is used for the manufacture of a medicament for the treatment of astrocytoma, wherein the medicament is to be used in combination with radiotherapy, wherein at least the specific integrin ligand cyclo-(Arg-Gly-Asp-DPhe-NMe-Val), the pharmaceutically acceptable solvates and/or salts thereof is to be administered 1 to 10 hours prior to the application of the radiotherapy, and wherein at least one cancer cotherapeutic agent comprising temozolomide is to be applied.

9. Use according to claim 1, wherein at least one specific integrin ligand, comprising cyclo-(Arg-Gly-Asp-DPhe-NMe-Val), is used for the manufacture of a medicament for the treatment of glioblastoma multiforme, wherein the medicament is to be used in combination with temozolomide and radiotherapy.

10. Use according to claim 8 and/or 9, wherein the radiotherapy is external beam radiation.

11. Use according to one or more of claims 1, 2, 3, 4, 5, 9 and 10, wherein at least the specific integrin ligand is to be applied 1 to 32 hours before the application of one or more further cancer cotherapeutic agents and/or radiotherapy.

12. Use according to claim 1, wherein at least one specific integrin ligand, comprising cyclo-(Arg-Gly-Asp-DPhe-NMe-Val), the pharmaceutically acceptable solvates and/or salts thereof, is used for the manufacture of a medicament for the treatment of astrocytoma, wherein the medicament is to be used in combination with temozolomide and radiotherapy, and wherein at least the specific integrin ligand cyclo-(Arg-Gly-Asp-DPhe-NMe-Val), the pharmaceutically acceptable solvates and/or salts thereof is to be administered to a patient in an amount of 800 mg to 7000 mg per week.

13. Use according to one or more of the proceeding claims, wherein cyclo-(Arg-Gly-Asp-DPhe-NMeVal) is to be administered to a patient in an amount of about 1000 mg per week, about 1500 mg per week, about 2500 mg per week, about 4000 mg per week or about 6000 mg per week.

14. Use according to one or more of the preceeding claims, wherein the amount of about 1000 mg per week or about 4000 mg per week is to be administered in a twice weekly administration scheme, and the amount of about 1500 mg per week or about 6000 mg per week is to be administered in a three times weekly administration scheme.

15. Use according to one or more of the preceeding claims, wherein the amount of about 1000 mg per week is to be administered in a twice weekly administration scheme consisting of about 500 mg per administration or the amount of about 4000 mg per week is to be administered in a twice weekly administration scheme of about 2000 mg per administration.

16. Use according to one or more of the preceeding claims, wherein at least the specific integrin ligand cyclo-(Arg-Gly-Asp-DPhe-NMe-Val), the pharmaceutically acceptable solvates and/or salts thereof is to be administered 2 to 8 hours or 3 to 8 hours prior to the application of the radiotherapy.

17. Use according to one or more of the claims 8 and 10 to 16, wherein additionally a further cancer cotherapeutic agent is to be applied, selected from Cisplatin, Oxaliplatin, Carboplatin, 5-FU, Darcabarzine, Procarbazine, Vinblastin, Vincristine, Irinotecan, Taxol, Paclitaxel, Docetaxel, Gemcitabine, Gleevec, Iressa, Tarceva and Nexavar, Herceptin, Bevacizumab, Cetuximab, Nimotuzumab, Sorafenib, Sunitinib and ZD6474 (ZACTIMA™), or selected from Cisplatin, Oxaliplatin, Vinblastin, Taxol, Gemcitabine, Gleevec and Iressa.

18. Use according to one or more of the preceeding claims, wherein the medicament is to be used in the treatment of patients having an increased DNA methylation status.

19. Use according to one or more of the preceeding claims, wherein the medicament is to be used in the treatment of patients showing partial or complete methylation of at least one promotor of at least one MGMT gene.

20. Use according to one or more of the preceeding claims, wherein the medicament is to be used in the treatment of newly diagnosed cancer, preferably in a first line treatment setting.

21. Cyclo-(Arg-Gly-Asp-DPhe-NMe-Val) for use for the treatment of astrocytoma in combination with temozolomide and radiotherapy.

22. Cyclo-(Arg-Gly-Asp-DPhe-NMe-Val) for use according to claim 21, wherein the astrocytoma is glioblastoma or glioblastoma multiforme.

23. Cyclo-(Arg-Gly-Asp-DPhe-NMe-Val) for use according to claim 21 and/or 22, wherein the radiotherapy is fractionated external beam radiation.

24. Cyclo-(Arg-Gly-Asp-DPhe-NMe-Val) for use according to claim 21, 22 and/or 23, wherein said Cyclo-(Arg-Gly-Asp-DPhe-NMe-Val) is to be applied substantially concurrently or sequentially to the temozolomide and/or the radiotherapy.

25. Cyclo-(Arg-Gly-Asp-DPhe-NMe-Val) for use according to claim 21, 22, 23 and/or 24, wherein said Cyclo-(Arg-Gly-Asp-DPhe-NMe-Val) is to be administered 1 to 8 hours (h) prior to the application of the radiotherapy and/or the temozolomide.

26. Cyclo-(Arg-Gly-Asp-DPhe-NMe-Val) for use according to claim 21, 22, 23, 24 and/or 25, wherein said Cyclo-(Arg-Gly-Asp-DPhe-NMe-Val) is to be administered 2 to 6 hours prior to the application of the radiotherapy and/or the temozolomide.

**Patentansprüche**

1. Verwendung von mindestens einem spezifischen Integrinligand, ausgewählt aus Cyclo-(Arg-Gly-Asp-DPhe-NMe-Val) und den pharmazeutisch verträglichen Solvaten und Salzen davon,
zur Herstellung eines Medikamentes zur Behandlung von Astrozytom, wobei das Medikament in Kombination mit Temozolomid und Strahlentherapie verwendet wird.

2. Verwendung nach Anspruch 1, wobei das Astrozytom Glioblastom oder Glioblastoma multiforme ist.

3. Verwendung nach Anspruch 1 und/oder 2, wobei die Strahlentherapie fraktionierte externe Strahlentherapie ist.

4. Verwendung nach einem oder mehreren der Ansprüche 1 bis 3, wobei der spezifische Integrinligand im Wesentlichen gleichzeitig mit dem Temozolomid und/oder der Strahlentherapie oder danach verabreicht werden soll.

5. Verwendung nach einem oder mehreren der Ansprüche 1 bis 4, wobei der mindestens eine Integrinligand Cyclo-(Arg-Gly-Asp-DPhe-NMeVal) ist.

6. Verwendung nach einem oder mehreren der Ansprüche 1 bis 5, wobei mindestens einer der spezifischen Integrinliganden 1 bis 8 Stunden (Std.) vor der Anwendung der Strahlentherapie und/oder des Temozolomids verabreicht werden soll.

7. Verwendung nach einem oder mehreren der Ansprüche 1 bis 6, wobei mindestens einer der spezifischen Integrinliganden 2 bis 6 Stunden vor der Anwendung der Strahlentherapie und/oder des Temozolomids verabreicht werden soll.

8. Verwendung nach Anspruch 1, wobei mindestens ein spezifischer Integrinligand, umfassend Cyclo-(Arg-Gly-Asp-DPhe-NMeVal), die pharmazeutisch verträglichen Solvate und/oder Salze davon, bei der Herstellung eines Medikamentes zur Behandlung von Astrozytom verwendet werden, wobei das Medikament in Kombination mit Strahlentherapie verwendet werden soll, wobei mindestens der spezifische Integrinligand Cyclo-(Arg-Gly-Asp-DPhe-NMeVal), die pharmazeutisch verträglichen Solvate und/oder Salze davon 1 bis 10 Std. vor der Anwendung der Strahlentherapie verabreicht werden soll(en), und wobei mindestens ein Krebs-Cotherapeutikum, das Temozolomid umfasst, verabreicht werden soll.

9. Verwendung nach Anspruch 1, wobei mindestens ein spezifischer Integrinligand, der Cyclo-(Arg-Gly-Asp-DPhe-NMeVal) umfasst, zur Herstellung eines Medikamentes zur Behandlung von Glioblastoma multiforme verwendet wird, wobei das Medikament in Kombination mit Temozolomid und Strahlentherapie verwendet werden soll.

10. Verwendung nach Anspruch 8 und/oder 9, wobei die Strahlentherapie externe Strahlentherapie ist.

11. Verwendung nach einem oder mehreren der Ansprüche 1, 2, 3, 4, 5, 9 und 10, wobei mindestens der spezifische Integrinligand 1 bis 32 Stunden vor der Anwendung von einem oder mehreren Krebs-Cotherapeutika und/oder Strahlentherapie verwendet werden soll.

12. Verwendung nach Anspruch 1, wobei mindestens ein spezifischer Integrinligand, umfassend Cyclo-(Arg-Gly-Asp-DPhe-NMeVal), die pharmazeutisch verträglichen Solvate und/oder Salze davon, zur Herstellung eines Medikamentes zur Behandlung von Astrozytom verwendet wird, wobei das Medikament in Kombination mit Temozolomid und Strahlentherapie verwendet werden soll, und wobei mindestens der spezifische Integrinligand Cyclo-(Arg-Gly-Asp-DPhe-NMeVal), die pharmazeutisch verträglichen Solvate und/oder Salze davon, einem Patient in einer Menge von 800 mg bis 7000 mg pro Woche verabreicht werden soll(en).

13. Verwendung nach einem oder mehreren der vorhergehenden Ansprüche, wobei Cyclo-(Arg-Gly-Asp-DPhe-NMeVal) einem Patient in einer Menge von etwa 1000 mg pro Woche, etwa 1500 mg pro Woche, etwa 2500 mg pro Woche, etwa 4000 mg pro Woche oder etwa 6000 mg pro Woche verabreicht werden soll.

**14.** Verwendung nach einem der vorhergehenden Ansprüche, wobei die Menge von etwa 1000 mg pro Woche oder etwa 4000 mg pro Woche in einem zweimal wöchentlichen Verabreichungsschema verabreicht werden soll, und die Menge von etwa 1500 mg pro Woche oder etwa 6000 mg pro Woche in einem dreimal wöchentlichen Verabreichungsschema verabreicht werden soll.

**15.** Verwendung nach einem oder mehreren der vorhergehenden Ansprüche, wobei die Menge von etwa 1000 mg pro Woche in einem zweimal wöchentlichen Verabreichungsschema, bestehend aus etwa 500 mg pro Verabreichung, verabreicht werden soll, oder die Menge von etwa 4000 mg pro Woche in einem zweimal wöchentlichen Verabreichungsschema von etwa 2000 mg pro Verabreichung, verabreicht werden soll.

**16.** Verwendung nach einem oder mehreren der vorhergehenden Ansprüche, wobei mindestens der spezifische Integrinligand Cyclo-(Arg-Gly-Asp-DPhe-NMeVal), die pharmazeutisch verträglichen Solvate und/oder Salze davon, 2 bis 8 Std. oder 3 bis 8 Std. vor der Anwendung der Strahlentherapie verabreicht werden soll(en).

**17.** Verwendung nach einem oder mehreren der Ansprüche 8 und 10 bis 16, wobei zudem ein weiteres Krebs-Cotherapeutikum, ausgewählt aus Cisplatin, Oxaliplatin, Carboplatin, 5-FU, Darcabarzin, Procarbazin, Vinblastin, Vincristin, Irinotecan, Taxol, Paclitaxel, Docetaxel, Gemcitabin, Gleevec, Iressa, Tarceva, und Nexavar, Herceptin, Bevacizumab, Cetuximab, Nimotuzumab, Sorafenib, Sunitib und ZD6474 (ZACTIMA™), oder ausgewählt aus Cisplatin, Oxaliplatin, Vinblastin, Taxol, Gemcitabin, Gleevec und Iressa, verabreicht werden soll.

**18.** Verwendung nach einem oder mehreren der vorhergehenden Ansprüche, wobei das Medikament bei der Behandlung von Patienten mit einem erhöhten DNA-Methylierungsstatus verwendet werden soll.

**19.** Verwendung nach einem oder mehreren der vorhergehenden Ansprüche, wobei das Medikament bei der Behandlung von Patienten verwenden soll, die eine partielle oder vollständige Methylierung von mindestens einem Promotor von mindestens einem MGMT-Gen verwendet werden soll.

**20.** Verwendung nach einem oder mehreren der vorhergehenden Ansprüche, wobei das Medikament bei der Behandlung von neu diagnostiziertem Krebs, vorzugsweise im Rahmen einer Primärbehandlung, verwendet werden soll.

**21.** Cyclo-(Arg-Gly-Asp-DPhe-NMeVal) zur Verwendung bei der Behandlung von Astrozytom in Kombination mit Temozolomid und Strahlentherapie.

**22.** Cyclo-(Arg-Gly-Asp-DPhe-NMeVal) zur Verwendung nach Anspruch 21, wobei das Astrozytom Glioblastom oder Glioblastome multiforme ist.

**23.** Cyclo-(Arg-Gly-Asp-DPhe-NMeVal) zur Verwendung nach Anspruch 21 und/oder 22, wobei die Strahlentherapie eine fraktionierte externe Strahlentherapie ist.

**24.** Cyclo-(Arg-Gly-Asp-DPhe-NMeVal) zur Verwendung nach Anspruch 21, 22 und/oder 23, wobei das Cyclo-(Arg-Gly-Asp-DPhe-NMeVal) im Wesentlichen gleichzeitig mit dem Temozolomid und/oder der Strahlentherapie oder danach angewendet werden soll.

**25.** Cyclo-(Arg-Gly-Asp-DPhe-NMeVal) zur Verwendung nach Anspruch 21, 22, 23 und/oder 24, wobei das Cyclo-(Arg-Gly-Asp-DPhe-NMeVal) 1 bis 8 Stunden (Std.) vor der Anwendung der Strahlentherapie und/oder des Temozolomids verabreicht werden soll.

**26.** Cyclo-(Arg-Gly-Asp-DPhe-NMeVal) zur Verwendung nach einem der Ansprüche 21, 22, 23, 24 und/oder 25, wobei das Cyclo-(Arg-Gly-Asp-DPhe-NMeVal) 2 bis 6 Std. vor der Anwendung der Strahlentherapie und/oder des Temozolomids verabreicht werden soll.


**Revendications**

**1.** Utilisation d'au moins un ligand des intégrines spécifique, choisi parmi le cyclo-(Arg-Gly-Asp-DPhe-NMeVal) et les solvats et sels pharmaceutiquement acceptables de celui-ci,
pour l'élaboration d'un médicament destiné au traitement de l'astrocytome, où le médicament est destiné à être utilisé en combinaison avec du témozolomide et une radiothérapie.

**2.** Utilisation selon la revendication 1, dans laquelle l'astrocytome est le glioblastome ou le glioblastome multiforme.

**3.** Utilisation selon la revendication 1 et/ou 2, dans laquelle la radiothérapie est une irradiation par faisceaux externes fractionnés.

**4.** Utilisation selon l'une ou plusieurs parmi les revendications 1 à 3, dans laquelle ledit ligand des intégrines spécifique est destiné à être appliqué essentiellement simultanément ou séquentiellement par rapport au témozolomide et/ou à la radiothérapie.

**5.** Utilisation selon l'une ou plusieurs parmi les revendications 1 à 4, dans laquelle le au moins un ligand des intégrines est le cyclo-(Arg-Gly-Asp-DPhe-NMeVal).

**6.** Utilisation selon l'une ou plusieurs parmi les revendications 1 à 5, dans laquelle au moins un parmi les ligands des intégrines spécifiques est destiné à être administré de 1 à 8 heures (h) préalablement à l'application de la radiothérapie et/ou du témozolomide.

**7.** Utilisation selon l'une ou plusieurs parmi les revendications 1 à 6, dans laquelle au moins un parmi les ligands des intégrines spécifiques est destiné à être administré de 2 à 6 heures préalablement à l'application de la radiothérapie et/ou du témozolomide.

**8.** Utilisation selon la revendication 1, dans laquelle au moins un ligand des intégrines spécifique, comprenant du cyclo-(Arg-Gly-Asp-DPhe-NMeVal), les solvats et/ou sels pharmaceutiquement acceptables de celui-ci, sont utilisés pour l'élaboration d'un médicament destiné au traitement de l'astrocytome, où le médicament est destiné à être utilisé en combinaison avec une radiothérapie, où au moins le ligand des intégrines spécifique, le cyclo-(Arg-Gly-Asp-DPhe-NMeVal), les solvats et/ou sels pharmaceutiquement acceptables de celui-ci, sont destinés à être administrés de 1 à 10 heures préalablement à l'application de la radiothérapie, et où au moins un agent co-thérapeutique anticancéreux comprenant du témozolomide, est destiné à être appliqué.

**9.** Utilisation selon la revendication 1, dans laquelle au moins un ligand des intégrines spécifique, comprenant du cyclo-(Arg-Gly-Asp-DPhe-NMeVal) est utilisé pour l'élaboration d'un médicament destiné au traitement du glioblastome multiforme, où le médicament est destiné à être utilisé en combinaison avec du témozolomide et une radiothérapie.

**10.** Utilisation selon la revendication 8 et/ou 9, dans laquelle la radiothérapie est une irradiation par faisceaux externes.

**11.** Utilisation selon l'une ou plusieurs parmi les revendications 1, 2, 3, 4, 5, 9 et 10, dans laquelle au moins le ligand des intégrines spécifique est destiné à être appliqué de 1 à 32 heures avant l'application d'un ou plusieurs autres agents co-thérapeutiques anticancéreux et/ou d'une radiothérapie.

**12.** Utilisation selon la revendication 1, dans laquelle au moins un ligand des intégrines spécifique, comprenant du cyclo-(Arg-Gly-Asp-DPhe-NMeVal), les solvats et/ou sels pharmaceutiquement acceptables de celui-ci, sont utilisés pour l'élaboration d'un médicament destiné au traitement de l'astrocytome, où le médicament est destiné à être utilisé en combinaison avec du témozolomide et une radiothérapie, et où au moins le ligand des intégrines spécifique, le cyclo-(Arg-Gly-Asp-DPhe-NMeVal), les solvats et/ou sels pharmaceutiquement acceptables de celui-ci, sont destinés à être administrés à un patient selon une quantité allant de 800 mg à 7000 mg par semaine.

**13.** Utilisation selon l'une ou plusieurs parmi les revendications précédentes, dans laquelle le cyclo-(Arg-Gly-Asp-DPhe-NMeVal) est destiné à être administré à un patient selon une quantité d'environ 1000 mg par semaine, d'environ 1500 mg par semaine, d'environ 2500 mg par semaine, d'environ 4000 mg par semaine, ou d'environ 6000 mg par semaine.

**14.** Utilisation selon l'une ou plusieurs parmi les revendications précédentes, dans laquelle la quantité d'environ 1000 mg par semaine ou d'environ 4000 mg par semaine est destinée à être administrée selon un schéma d'administration de deux fois par semaine, et la quantité d'environ 1500 mg par semaine ou d'environ 6000 mg par semaine est destinée à être administrée selon un schéma d'administration de trois fois par semaine.

**15.** Utilisation selon l'une ou plusieurs parmi les revendications précédentes, dans laquelle la quantité d'environ 1000 mg par semaine est destinée à être administrée selon un schéma d'administration de deux fois par semaine constitué d'environ 500 mg par administration, ou la quantité d'environ 4000 mg par semaine est destinée à être administrée selon un schéma d'administration de deux fois par semaine d'environ 2000 mg par administration.

16. Utilisation selon l'une ou plusieurs parmi les revendications précédentes, dans laquelle au moins le ligand des intégrines spécifique, le cyclo-(Arg-Gly-Asp-DPhe-NMeVal), les solvats et/ou sels pharmaceutiquement acceptables de celui-ci, sont destinés à être administrés de 2 à 8 heures ou de 3 à 8 heures préalablement à l'application de la radiothérapie.

17. Utilisation selon l'une ou plusieurs parmi les revendications 8 et 10 à 16, dans laquelle, en outre, un autre agent co-thérapeutique anticancéreux est destiné à être administré, choisi parmi le cisplatine, l'oxaliplatine, le carboplatine, le 5-FU, la dacarbazine, la procarbazine, la vinblastine, la vincristine, l'irinotécan, le taxol, le paclitaxel, le docétaxel, la gemcitabine, le gleevec, l'iressa, la tarveca et le nexavar, l'herceptine, le bevacizumab, le cetuximab, le nimotuzumab, le sorafénib, le sunitinib et ZD6474 (ZACTIMA™), ou choisi parmi le cisplatine, l'oxaliplatine, la vinblastine, le taxol, la gemcitabine, le gleevec, et l'iressa.

18. Utilisation selon l'une ou plusieurs parmi les revendications précédentes, dans laquelle le médicament est destiné à être utilisé dans le traitement de patients ayant un état accru de méthylation de l'ADN.

19. Utilisation selon l'une ou plusieurs parmi les revendications précédentes, dans laquelle le médicament est destiné à être utilisé dans le traitement de patients présentent un méthylation partielle ou complète d'au moins un promoteur d'au moins un gène MGMT.

20. Utilisation selon l'une ou plusieurs parmi les revendications précédentes, dans laquelle le médicament est destiné à être utilisé dans le traitement de cancer nouvellement diagnostiqué, préférablement dans le cadre d'un traitement de première intention.

21. Cyclo-(Arg-Gly-Asp-DPhe-NMeVal) pour une utilisation dans le traitement de l'astrocytome en combinaison avec du témozolomide et une radiothérapie.

22. Cyclo-(Arg-Gly-Asp-DPhe-NMeVal) pour une utilisation selon la revendication 21, **caractérisé en ce que** l'astrocytome est un glioblastome ou un glioblastome multiforme.

23. Cyclo-(Arg-Gly-Asp-DPhe-NMeVal) pour une utilisation selon la revendication 21 et/ou 22, **caractérisé en ce que** la radiothérapie est une irradiation par faisceaux externes fractionnés.

24. Cyclo-(Arg-Gly-Asp-DPhe-NMeVal) pour une utilisation selon la revendication 21, 22 et/ou 23, **caractérisé en ce que** ledit cyclo-(Arg-Gly-Asp-DPhe-NMeVal) est destiné à être appliqué essentiellement simultanément ou séquentiellement par rapport au témozolomide et/ou à la radiothérapie.

25. Cyclo-(Arg-Gly-Asp-DPhe-NMeVal) pour une utilisation selon la revendication 21, 22, 23 et/ou 24, **caractérisé en ce que** ledit cyclo-(Arg-Gly-Asp-DPhe-NMeVal) est destiné à être administré de 1 à 8 heures (h) préalablement à l'application de la radiothérapie et/ou du témozolomide.

26. Cyclo-(Arg-Gly-Asp-DPhe-NMeVal) pour une utilisation selon la revendication 21, 22, 23, 24 et/ou 25, **caractérisé en ce que** ledit cyclo-(Arg-Gly-Asp-DPhe-NMeVal) est destiné à être administré de 2 à 6 heures préalablement à l'application de la radiothérapie et/ou du témozolomide.

**Figure 1**

Kaplan Meier survival curves of nude rats bearing orthotopic human glioblastomas U251 derived.

Dark square represents survival of non irradiated animals.

## Figure 2

## REFERENCES CITED IN THE DESCRIPTION

### Patent documents cited in the description

- US 4517686 A
- US 4578079 A
- US 4589881 A
- US 4614517 A
- US 4661111 A
- US 4792525 A
- EP 0770622 A
- US 5753230 A
- US 5766591 A
- WO 9532710 A
- WO 9537655 A
- WO 9701540 A
- WO 9737655 A
- WO 9745137 A
- WO 9741844 A
- WO 9808840 A
- WO 9818460 A
- WO 9818461 A
- WO 9825892 A
- WO 9831359 A
- WO 9830542 A
- WO 9915506 A
- WO 9915507 A
- WO 9931061 A
- WO 0006169 A
- EP 0853084 A
- EP 0854140 A
- EP 0854145 A
- US 5780426 A
- US 6048861 A
- WO 9600574 A
- WO 9600730 A
- WO 9606087 A
- WO 9626190 A
- WO 9724119 A
- WO 97124122 A
- WO 9724124 A
- WO 9815278 A
- WO 9905107 A
- WO 9906049 A
- WO 9915170 A
- WO 9915178 A
- WO 9734865 A
- WO 9911626 A
- WO 9915508 A
- WO 9930709 A
- WO 9930713 A
- WO 9931099 A
- WO 0009503 A
- US 5919792 A
- US 5925655 A
- US 5981546 A
- US 6017926 A
- WO 0072801 A
- WO 0038665 A
- WO 9745447 A
- WO 9937683 A
- US 4816567 A
- US 4816397 A
- US 5225539 A, Winter
- US 4342566 A
- WO 9316185 A
- US 5571894 A
- US 5587458 A
- US 4474893 A
- WO 9100360 A
- WO 9205793 A
- WO 9604305 A
- WO 9210209 A
- WO 9311162 A
- WO 9103493 A
- WO 9413804 A
- WO 9850431 A
- US 6773897 B
- US 5260291 A
- US 5786146 A
- US 6017704 A
- US 6200756 B
- US 6265171 B
- US 5817514 A, Li
- US 5407804 A

### Non-patent literature cited in the description

- **DAVIS et al.** *J. Cell. Biochem.,* 1993, vol. 51, 206
- **DAVIS et al.** *J. Cell. Biol.,* 1993, vol. 51, 206
- **CHERESH et al.** *Cell,* 1989, vol. 58, 945
- **AUMAILLEY et al.** *FEBS Letts,* 1991, vol. 291, 50

- Isolated liver perfusion. **K. R. AIGNER.** Hepatic Metastases. Butterworth Heinemann, 1996, 101-107
- **KEENAN et al.** *Bioorg. Med. Chem. Lett.,* 1998, vol. 8 (22), 3171
- **WARD et al.** *Drug Metab. Dispos,* 1999, vol. 27 (11), 1232
- **SMITH et al.** *J. Biol. Chem.,* 1990, vol. 265, 12267
- **BROOKS et al.** *Cell,* 1994, vol. 79, 1157
- **CHOTHIA ; LESK.** *J. Mol. Biol.,* 1987, vol. 196, 901-917
- **KOHLER ; MILSTEIN.** *Nature,* 1975, vol. 256, 495
- Monoclonal Antibody Technology, The Production and Characterization of Rodent and Human Hybridomas. Laboratory Techniques in Biochemistry and Molecular Biology. Elsevier Science Publishers, 1985, vol. 13
- **CLACKSON et al.** *Nature,* 1991, vol. 352, 624-628
- **MARKS et al.** *J. Mol. Biol.,* 1991, vol. 222 (58), 1-597
- **MORRISON et al.** *Proc. Nat. Acad. Sci., USA,* 1984, vol. 81, 6851-6855
- **HERMANSON.** Bioconjugate Techniques. Academic Press, 1996
- **PLÜCKTHUN.** The Pharmacology of Monoclonal Antibodies. Springer-Verlag, 1994, vol. 113, 269-315
- **HUSTON et al.** *Proc. Natl. Acad. Sci.,* 1988, vol. 85, 5879
- **SKERRA ; PLUECKTHUN.** *Science,* 1988, vol. 240, 1038
- **KRANZ et al.** *Proc. Natl. Acad. Sci. USA,* 1981, vol. 78, 5807
- **LANDSCHULZ et al.** *Science,* 1988, vol. 240, 1759
- **MANIATIS ; ABEL.** *Nature,* 1989, vol. 341, 24
- **O'SHEA et al.** *Science,* 1989, vol. 245, 646
- **KARPOVSKY et al.** *J. EXP. Med.,* 1984, vol. 160, 1686
- **LIU.** *Proc. Natl. Acad. Sci. USA,* 1985, vol. 82, 8648
- **PAULUS.** *Behring Inst. Mitt., No. 78,* 1985, 118
- **BRENNAN.** *Science,* 1985, vol. 30, 81
- **GLENNIE et al.** *J. Immunol.,* 1987, vol. 139, 2367
- **R.STUPP et al.** *NEJM,* 2005
- **HEGI et al.** *NEJM,* 2005, vol. 352, 997-1003
- **PALMISANO WA ; DIVINE KK ; SACCOMANNO G et al.** Predicting lung cancer by detecting aberrant promoter methylation in sputum. *Cancer Res,* 2000, vol. 60, 5954-8
- **AHRENDT et al.** *J. Natl. Cancer Inst.,* 1999, vol. 91, 332-339
- **BELSINKY et al.** *Proc. Natl. Acad. Sci. U.S.A.,* 1998, vol. 95, 11891-11896
- **CLARK et al.** *Nucleic Acids Res.,* 1994, vol. 22, 2990-2997
- **HERMAN et al.** *Proc Natl Acad Sd U.S.A.,* 1996, vol. 93, 9821-9826
- **XIONG ; LAIRD.** *Nucleic Acids Res.,* 1997, vol. 25, 2532-2534
- **EADS et al.** *Nuc. Acids. Res.,* 2002, vol. 28, 32
- **COTTRELL et al.** *Nucleic Acids Res.,* 2004, vol. 32, 1-8
- **HERMAN et al.** *Proc. Natl. Acad Sci USA,* 1996, vol. 93 (18), 9821-9826
- **ESTELLER et al.** *Cancer Res.,* 1999, vol. 59, 793-797
- **MYMES et al.** *Carcinogenesis,* 1984, vol. 5, 1061-1064
- **FUTSCHER et al.** *Cancer Comm.,* 1989, vol. 1, 65-73
- **KREKLAW et al.** *J. Pharmacol. Exper. Ther.,* 2001, vol. 297 (2), 524-530
- **NAGEL et al.** *Anal. Biochem.,* 2003, vol. 321 (1), 38-43
- **ENGEBRAATEN,O. ; HJORTLAND,G.O. ; HIRSCHBERG,H. ; FODSTAD,O.** Growth of precultured human glioma specimens in nude rat brain. *J. Neurosurg,* 1999, vol. 90, 125-132
- **KIM,J.H. ; KHIL,M.S. ; KOLOZSVARY,A. ; GUTIERREZ,J.A. ; BROWN,S.L.** Fractionated radiosurgery for 9L gliosarcoma in the rat brain. *Int. J. Radiat. Oncol. Biol. Phys.,* 1999, vol. 45, 1035-1040